# EUROPEAN PATENT APPLICATION

(11) **EP 4 792 808 A1**
(43) Date of publication of application: **19.08.2026**
(21) Application number: 24876537.2
(22) Date of filing: 09.10.2024
(51) Int. Cl.: A61K 31/18, A61K 9/48

(54) **PHARMACEUTICAL COMPOSITION CONTAINING MITOPHAGY INDUCER**

(30) Priority: 10.10.2023 CN 202311311012; 19.12.2023 CN 202311750733
(71) Applicant: Hangzhou Phecdamed Co., Ltd., Hangzhou, Zhejiang 310023 (CN)
(72) Inventor: LIU, Dong, Hangzhou, Zhejiang 310023 (CN); TIAN, Zhen, Hangzhou, Zhejiang 310023 (CN); WU, Ronghai, Hangzhou, Zhejiang 310023 (CN)
(74) Representative: Viering, Jentschura & Partner mbB Patent- und Rechtsanwälte
(86) International application number: PCT/CN2024/123604
(87) International publication number: WO 2025/077724

(57) **Abstract**

A pharmaceutical composition containing a mitophagy inducer as an active substance. The pharmaceutical composition is conducive to being prepared into a pharmaceutical preparation, can avoid incompatibility caused by the interaction between the active substance and an auxiliary material, and thus can be stored for a long period of time.

## Description

### TECHNICAL FIELD

The present invention relates to a pharmaceutical composition comprising a mitophagy inducer, which belongs to the field of pharmaceutical preparations.

### BACKGROUND TECHNOLOGY

A compound of Formula I and a specific compound thereof (particularly TJ0113) are described in CN115894404A, and are mitophagy inducers that can be used to inhibit or alleviate various acute or chronic diseases caused by mitophagy dysfunction. The structures of the general formula and compound TJ0113 are as follows:

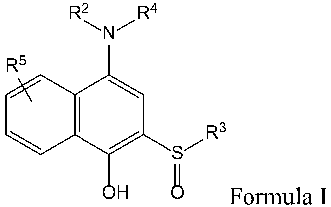

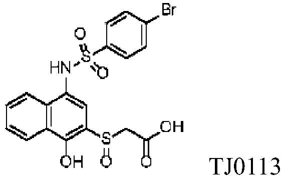

No prior art has conducted any research on pharmaceutical compositions or preparations of the compound. The inventors of the present application found that the mitophagy inducer compound is prone to stability problems when prepared into a preparation because the compound is prone to interact with a variety of auxiliary materials commonly used in the pharmaceutical industry, resulting in incompatibility, and is not conducive to long-term storage. Therefore, it is urgently necessary in the art to develop a pharmaceutical composition to solve the incompatibility problem between the mitophagy inducer compounds and auxiliary materials, and thus obtain a preparation that can be stored for a long period of time.

### SUMMARY OF INVENTION

The inventors of the present application have conducted extensive research to solve the aforementioned problems, thus developing a pharmaceutical composition containing a mitophagy inducer that can overcome the incompatibility problem. Specifically, the present invention includes but is not limited to the following technical contents:
1. A pharmaceutical composition, characterized in that the pharmaceutical composition contains a compound of Formula I or a pharmaceutically acceptable salt thereof, a diluent, a disintegrant, a lubricant, and does not contain any of the following ingredients: hydroxypropyl cellulose, crospovidone, colloidal silica, magnesium stearate, talc, mannitol, croscarmellose sodium, 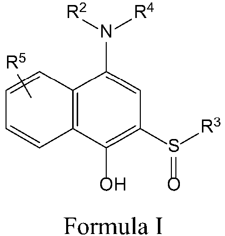
   wherein, R² is hydrogen, C_{1~4} alkyl, C_{3~6} cycloalkyl, four to six-membered epoxy alkyl;
   R³ is 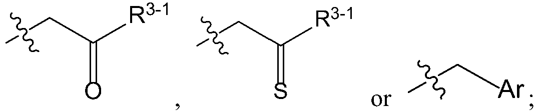
   wherein, R³⁻¹ is hydrogen, hydroxyl, C_{1~4} alkyl, C_{1~4} alkoxy, C_{3~6} cycloalkyl, three to six-membered epoxy alkyl, -N(R³⁻²R^{3-2a}), -CH₂C(O)R³⁻², -CH₂C(O)OR³⁻², -CH₂C(O)NR³⁻² R^{3-2a}
   R³⁻² and R^{3-2a} are independently hydrogen, C_{1~4} alkyl or three to six-membered cycloalkyl respectively,
   Ar is phenyl, 5 or 6-membered monocyclic heteroaryl, 5 or 6-membered monocyclic heteroaryl substituted by at least one R³⁻³, the R³⁻³ is hydrogen, halogen, C_{1~4} alkyl, three to six-membered cycloalkyl, hydroxyl, C_{1~4} Alkoxy, three to six-membered epoxy alkyl, C_{1~4} halogenated alkyl, C_{2~4} alkenyl, C2~4 alkynyl, -N(R^{3-3a}R^{3-3b}) or phenyl,
   R^{3-3a} and R^{3-3b} are independently hydrogen, C_{1~4} alkyl or three to six-membered cycloalkyl respectively;
   R⁴ is 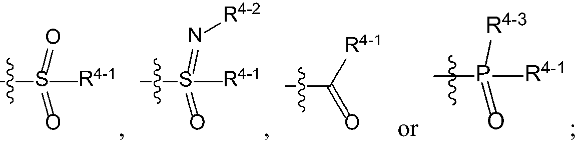
   wherein, R⁴⁻¹ is phenyl, phenyl substituted by at least one R⁴⁻¹¹, 5 or 6-membered monocyclic heteroaryl, 5 or 6-membered monocyclic heteroaryl substituted by at least one R⁴⁻¹¹, 8 to 10-membered fused bicyclic heteroaryl, 8 to 10-membered fused bicyclic heteroaryl substituted by at least one R⁴⁻¹¹, the R⁴⁻¹¹ is halogen, nitro, C_{1~4} alkyl, C_{3~6} cycloalkyl, C_{1~4} alkoxy, -N(R^{4-1a}R^{4-1b}), phenyl, C_{1~4} halogenated alkyl, C_{1~4} halogenated alkoxy or 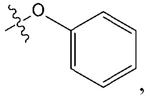 R^{4-1a} and R^{4-1b} are independently hydrogen, C_{1~4} alkyl or C_{3~6} cycloalkyl respectively, and R^{4-1a} and R^{4-1b} may be bonded to each other to form a ring,
   R⁴⁻² is C_{1~4} alkyl, C_{3~6} cycloalkyl, or when R² is C_{1~4} alkyl, R⁴⁻² is bonded to R² to form a 4~8-membered ring,
   R⁴⁻³ is C_{1~4} alkyl or C_{1~4} alkoxy;
   the number of R⁵ is 0~5, when the number of R⁵ is not 0, each R⁵ is independently selected from halogen, nitro, nitrile, -N⁺(R⁵⁻¹)₃, C_{1~4} halogenated alkyl, -C(O)OR⁵⁻¹, -C(O)R⁵⁻¹, -C(O)N(R⁵⁻¹R^{5-1a}), -C(O)N(R⁵⁻¹R^{5-1a}), -S(O)₂R⁵⁻¹, -S(O)R⁵⁻¹, -N=C(R⁵⁻¹R^{5-1a}), hydroxyl, C_{1~4} alkyl, phenyl, phenyl with at least one hydrogen substituted by R⁵⁻¹, C_{1~4} alkoxy, -N(R⁵⁻¹R^{5-1a}), -N(R⁵⁻¹)C(O)R^{5-1a}, -OC(O)R⁵⁻¹, -OC(O) N(R⁵⁻¹R^{5-1a}) or -SR⁵⁻¹, wherein R⁵⁻¹, R^{5-1a} and R^{5-1b} are independently hydrogen, C_{1~4} alkyl, C_{2~4} alkenyl, C_{2~4} alkynyl, C_{1~4} alkyl with at least one hydrogen substituted by halogen, C_{2~4} alkenyl with at least one hydrogen substituted by halogen or C_{2~4} alkynyl with at least one hydrogen substituted by halogen respectively.
2. The pharmaceutical composition according to technical content 1, wherein the diluent comprises microcrystalline cellulose and pregelatinized starch.
3. The pharmaceutical composition according to technical content 1 or 2, wherein the disintegrant is sodium carboxymethyl starch.
4. The pharmaceutical composition according to any one of technical contents 1-3, wherein the lubricant is sodium stearyl fumarate.
5. The pharmaceutical composition according to any one of technical contents 1-4, wherein R² is hydrogen or C_{1~4} alkyl;
   R³ is 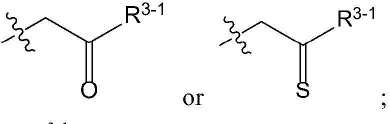
   wherein, R³⁻¹ is hydrogen, hydroxyl, C_{1~4} alkyl or C_{1~4} alkoxy,
   R⁴ is 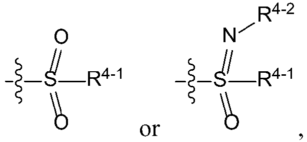
   wherein, R⁴⁻¹ is phenyl, phenyl substituted by at least one R⁴⁻¹¹, 5 or 6-membered monocyclic heteroaryl, 5 or 6-membered monocyclic heteroaryl substituted by at least one R⁴⁻¹¹, 8 to 10-membered fused bicyclic heteroaryl, 8 to 10-membered fused bicyclic heteroaryl substituted by at least one R⁴⁻¹¹, and the R⁴⁻¹¹ is Halogen, nitro, C_{1~4} alkyl, C_{3~6} cycloalkyl, C_{1~4} alkoxy, -N(R^{4-1a}R^{4-1b}), phenyl, C_{1~4} halogenated alkyl, C_{1~4} halogenated alkoxy or 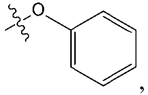 R^{4-1a} and R^{4-1b} is independently hydrogen, C_{1~4} alkyl or C_{3~6} cycloalkyl respectively, and R^{4-1a} and R^{4-1b} may be bonded to each other to form a ring,
   R⁴⁻² is C_{1~4} alkyl, C_{3~6} cycloalkyl, or when R² is C_{1~4} alkyl, R⁴⁻² is bonded to R² to form a 4-8-membered ring,
   R⁴⁻³ is C_{1~4} alkyl or C_{1~4} alkoxy;
   the number of R⁵ is 0~5, when the number of R⁵ is not 0, each R⁵ is independently selected from halogen, nitro, nitrile, -N⁺(R⁵⁻¹)₃, C_{1~4} halogenated alkyl, -C(O)OR⁵⁻¹, -C(O)R⁵⁻¹, -C(O)N(R⁵⁻¹R^{5-1a}), -C(O)N(R⁵⁻¹R^{5-1a}), -S(O)₂R⁵⁻¹, -S(O)R⁵⁻¹, -N=C(R⁵⁻¹R^{5-1a}), hydroxyl, C_{1~4} alkyl, phenyl, phenyl with at least one hydrogen substituted by R⁵⁻¹, C_{1~4} alkoxy, -N(R⁵⁻¹R^{5-1a}), -N(R⁵⁻¹)C(O)R^{5-1a}, -OC(O)R⁵⁻¹, -OC(O) N(R⁵⁻¹R^{5-1a}) or -SR⁵⁻¹, wherein R⁵⁻¹, R^{5-1a} and R^{5-1b} are independently hydrogen, C_{1~4} alkyl, C_{2~4} alkenyl, C_{2~4} alkynyl, C_{1~4} alkyl with at least one hydrogen substituted by halogen, C_{2~4} alkenyl with at least one hydrogen substituted by halogen or C_{2~4} alkynyl with at least one hydrogen substituted by halogen respectively.
6. The pharmaceutical composition according to technical content 5, wherein R² is hydrogen or C1~4 alkyl,
   R³ is 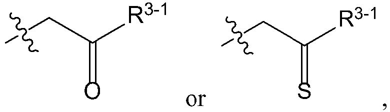 R³⁻¹ is hydroxy or C1~4 alkoxy,
   R⁴ is 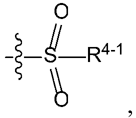
   wherein, R⁴⁻¹ is phenyl, phenyl substituted by at least one R⁴⁻¹¹, 5 or 6-membered monocyclic heteroaryl, or 5 or 6-membered monocyclic heteroaryl substituted by at least one R⁴⁻¹¹, the R⁴⁻¹¹ is halogen, nitro, C_{1~4} alkyl, C_{3~6} Cycloalkyl, C_{1~4} alkoxy or C_{1~4} halogenated alkyl; and
   the number of R⁵ is 0.
7. The pharmaceutical composition according to technical content 6, wherein R² is hydrogen,
   R³ is 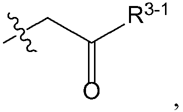 R³⁻¹ is hydroxyl,
   R⁴ is 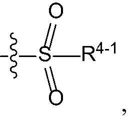
   wherein, R⁴⁻¹ is phenyl substituted by at least one R⁴⁻¹¹, the R⁴⁻¹¹ is halogen, C_{1~4} alkyl, C_{1~4} alkoxy or C_{1~4} halogenated alkyl; and
   the number of R⁵ is 0.
8. The pharmaceutical composition according to technical content 7, wherein the compound of Formula I is TJ0113. 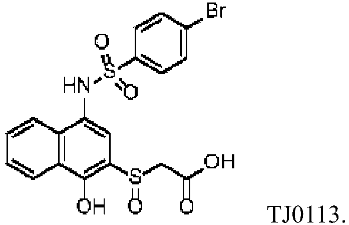
9. The pharmaceutical composition according to technical content 8, wherein the TJ0113 is in a solid form of a sodium salt represented by the following formula: 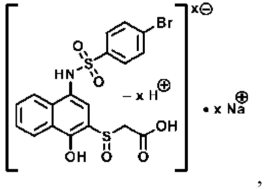 wherein, X is 0.1-2, for example 1.
10. The pharmaceutical composition according to technical content 9, wherein the X-ray powder diffraction pattern of the solid form exhibits characteristic peaks (Cu Kα rays) at the following 2θ angles: 7.06° (±0.2°) and 20.87° (±0.2°).
11. The pharmaceutical composition according to any one of technical contents 1-10, wherein the pharmaceutical composition contains compound TJ0113 or a pharmaceutically acceptable salt thereof, microcrystalline cellulose, pregelatinized starch, sodium carboxymethyl starch, sodium stearyl fumarate .
12. The pharmaceutical composition according to technical content 11, wherein the pharmaceutical composition consists of compound TJ0113 or a pharmaceutically acceptable salt thereof, microcrystalline cellulose, pregelatinized starch, sodium carboxymethyl starch, sodium stearyl fumarate.
13. The pharmaceutical composition according to any one of technical contents 1-12, wherein a content percentage of the compound of Formula I or the pharmaceutically acceptable salt thereof in the pharmaceutical composition is 1.0% to 50% by weight, a content percentage of diluent in the pharmaceutical composition is 30% to 90% by weight, a content percentage of disintegrant in the pharmaceutical composition is 0.1% to 25% by weight, and a content percentage of lubricant in the pharmaceutical composition is 0.01% to 5.0% by weight.
14. A pharmaceutical preparation containing a pharmaceutical composition according to any one of technical contents 1-13, wherein the pharmaceutical preparation is selected from tablets, capsules, granules, powders, and pills.
15. The pharmaceutical preparation according to technical content 14, wherein the pharmaceutical preparation is a capsule, which consists of a capsule shell and a content, the content is a pharmaceutical composition according to any one of technical contents 1-13.

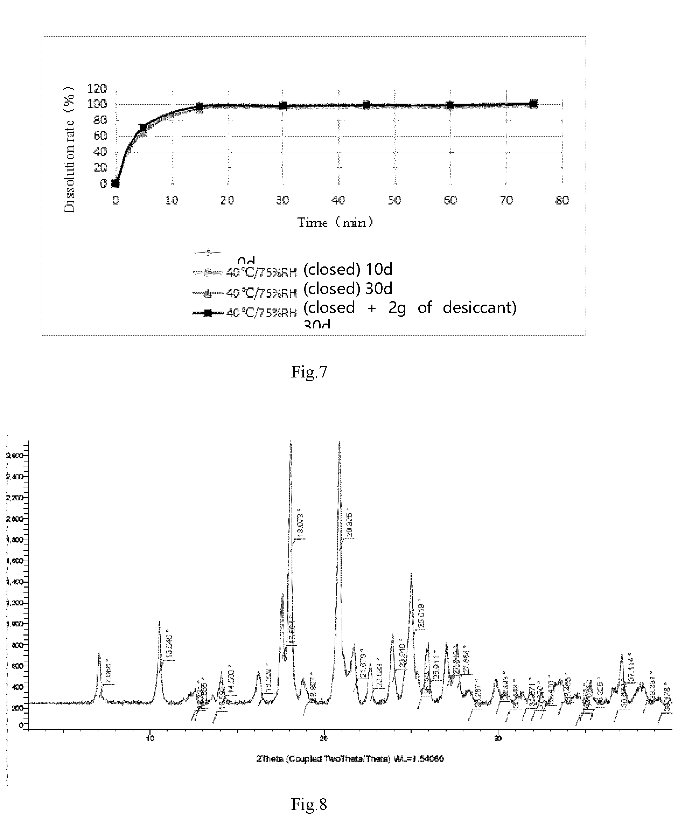

Preferably, in the aforementioned technical solution involving solid form of sodium salt of compound TJ0113, its X-ray powder diffraction pattern exhibits at least one characteristic peak at the 2θ angles selected from 7.06° (±0.2°), 18.07° (±0.2°), 25.02° (±0.2°), 17.58° (±0.2°), 20.87° (±0.2°), 10.54° (±0.2°), 23.91° (±0.2°). More preferably, the X-ray powder diffraction pattern of the solid form of the sodium salt of the compound of Formula (I) exhibits at least one (more preferably at least two, most preferably at least three) characteristic peaks at the 2θ angles selected from 7.06° (±0.2°), 18.07° (±0.2°), 25.02° (±0.2°), 17.58° (±0.2°), 20.87° (±0.2°), 10.54° (±0.2°), 23.91° (±0.2°), 27.65° (±0.2°), 27.05° (±0.2°), 21.68° (±0.2°), 25.91° (±0.2°). More preferably, the solid form of the sodium salt of the compound of Formula (I) (also referred to herein as "D crystal form") exhibits an X-ray powder diffraction pattern that is substantially the same as in FIG. 8.

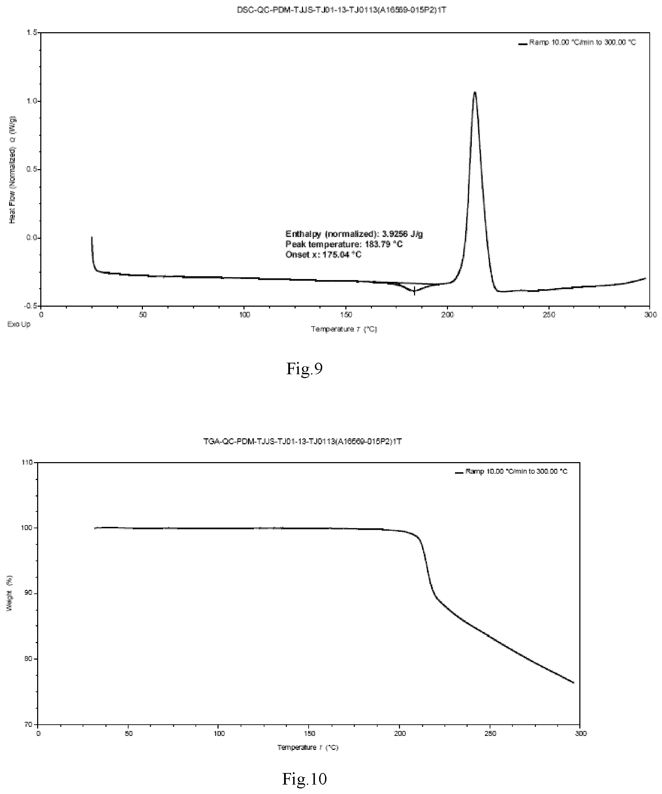

As the D crystal solid form of the sodium salt of the compound TJ0113, its differential scanning calorimetry curve has an endothermic peak at 183.79°C (±3°C) and an exothermic peak at 210.79°C (±3°C). As a solid form of the sodium salt of the compound of Formula (I) of the present invention, the thermogravimetric analysis curve shows a thermogravimetric analysis curve (DSC) pattern that is substantially the same as in FIG. 9.

In some preferred embodiments, the thermogravimetric analysis curve of the D crystal solid form of the sodium salt of the compound TJ0113 shows a thermogravimetric analysis curve pattern that is substantially the same as in FIG. 10.

In some preferred embodiments, the pharmaceutical composition comprises the following ingredients by weight parts:
Compound of Formula (I) or a pharmaceutically acceptable salts thereof...20-30 parts;
Diluent...50-80 parts;
Disintegrant...1-10 parts;
Lubricant...0.1-3 parts.

In some preferred embodiments, the pharmaceutical composition comprises the following ingredients by weight parts:
Compound of Formula (I) or a pharmaceutically acceptable salt thereof...23-28 parts;
Diluent...60-70 parts;
Disintegrant...3-8 parts;
Lubricant...0.1-1 parts.

In some preferred embodiments, the diluent is selected from at least one of microcrystalline cellulose, lactose, mannitol, starch and dextrin.

In some preferred embodiments, the disintegrant is selected from at least one of sodium carboxymethyl starch, sodium croscarmellose, and crospovidone.

In some preferred embodiments, the lubricant is selected from at least one of magnesium stearate, sodium stearic fumarate, and talc.

In some preferred embodiments, the pharmaceutical composition may further comprise thickeners (e.g., gelatin, arabic gelatin), preservatives (e.g., benzoate, sorbate), adhesives (e.g., mixtures of powdered sugar and syrup, polyvinyl pyrrolidone), sweeteners (e.g., sucrose, aspartame), stabilizers (e.g., ascorbic acid, sodium thiosulfate), and/or glidants (e.g., colloidal silica, talc).

In some preferred embodiments, the diluent is microcrystalline cellulose and pregelatinized starch.

In some preferred embodiments, the disintegrant is sodium carboxymethyl starch.

In some preferred embodiments, the lubricant is sodium stearyl fumarate.

### BRIEF DESCRIPTION OF THE DRAWINGS

Fig. 1 shows the effect of the diluent type on the dissolution result in Example 2 (paddle method + basket sinker, 50 rpm).
Fig. 2 shows the effect of the diluent ratio on the dissolution result in Example 2 (basket method, 75 rpm).
Fig. 3 shows the effect of the disintegrant type on the dissolution result in Example 3 (basket method, 75 rpm).
Fig. 4 shows the effect of the solubilizer on the dissolution result in Example 3 (basket method, 75 rpm).
Fig. 5 shows the dissolution experiment result of 20 mg capsule in Example 3 (paddle method + basket sinker, 75 rpm).
Fig. 6 shows the dissolution result of 20 mg capsule of F14 in Example 4 (paddle method + basket sinker, 75 rpm).
Fig. 7 shows the stability dissolution result of 100 mg capsule of F13 in Example 4 (paddle method + basket sinker, 75 rpm).
Fig. 8 shows the X-ray powder diffraction pattern of the D crystal form of the sodium salt of compound TJ0113.
Fig. 9 shows a differential scanning calorimetric curve pattern of the D crystal form of the sodium salt of compound TJ0113.
Fig. 10 shows the thermogravimetric analysis curve pattern of the D crystal form of the sodium salt of compound TJ0113.
Fig. 11 shows the XRPD pattern of the sample before and after dry granulation in Example 5.

### DETAILED DESCRIPTION OF EMBODIMENTS

A compound of Formula I or a pharmaceutically acceptable salt thereof (such as compound TJ0113) is also referred to herein as "active ingredient" or "API". Those skilled in the art know that compounds of Formula I have similar properties due to their similar structures, and therefore, compounds within their scope are applicable to preparations defined in the present invention.

The inventors of the present application found that there was an incompatibility problem between the active ingredient and the auxiliary materials such as hydroxypropyl cellulose, crospovidone, colloidal silica, magnesium stearate, talc, sugar alcohol (especially mannitol), and croscarmellose sodium, the detail refers to the experimental contents of the Examples section. Therefore, the preparation of the present invention does not contain these auxiliary materials with compatibility problems.

In the pharmaceutical composition of the present invention, the content percentage of the active ingredient in the pharmaceutical composition may be, for example, 1.0 wt% ~50 wt%, such as 1.0 wt%, 2.0 wt%, 5.0 wt%, 8.0 wt%, 10.0 wt%, 12 wt%, 15 wt% ~ 25 wt%, 30 wt%, 35 wt%, 40 wt%, 50 wt%, for example, may be 10.0 wt% ~ 40 wt%, or 16 wt%~30 wt%, preferably 20 wt% ~ 25 wt%, or about 25 wt%.

A diluent refers to an auxiliary material used to increase the volume or weight of a solid pharmaceutical preparation, also known as a filler. Except for the aforementioned auxiliary materials with compatibility problems, the pharmaceutical compositions of the present invention may contain diluents known in the art, such as lactose, microcrystalline cellulose and pregelatinized starch. In order to speed up dissolution, a mixture of microcrystalline cellulose and pregelatinized starch is preferred; as verified by the experiments in the embodiments, the ratio of the two has little effect on the dissolution result, for example, it may be 5:1-0.5:1, preferably 3:1-1:1. The content percentage of the diluent in the pharmaceutical composition may be, for example, 30 wt% ~90 wt%, such as 30 wt%, 35 wt%, 40 wt%, 50 wt%, 55 wt%, 60 wt%, 65 wt%, 70 wt% to 75 wt%, 80 wt%, 85 wt%, 90 wt%, for example, may be 40 wt% ~ 85 wt%, or 55 wt% ~ 80 wt%, preferably 65 wt%~75 wt%, or about 70 wt%, wherein the content percentage of the pregelatinized starch in the pharmaceutical composition may be, for example, 1.0 wt% ~ 50 wt%, such as 1.0 wt%, 2.0 wt%, 5.0 wt%, 8.0 wt%, 10.0 wt%, 12 wt%, 15 wt% ~ 25 wt%, 30 wt%, 35 wt%, 40 wt%, 50 wt%, for example, may be 10.0 wt% ~ 30 wt%, or 12 wt% ~ 30 wt%, preferably 15 wt% ~ 25 wt%. The diluents used in the present application are, for example, commercially available microcrystalline cellulose 102 (available from Microcellulose Weissenborn GmbH+Co.KG) and pregelatinized starch STARCH 1500 ^{®} (available from Colorcon).

A disintegrant refers to an auxiliary material used to promote the rapid fragmentation of solid preparations into fine particles in gastrointestinal fluid. Except for the aforementioned auxiliary materials with compatibility problems, the pharmaceutical compositions of the present invention may contain disintegrants known in the art, such as sodium carboxymethyl starch. The content percentage of the disintegrant in the pharmaceutical composition may be, for example, 0.1 wt% ~ 25 wt%, such as 0.1 wt%, 0.5 wt%, 1.0 wt%, 1.5 wt%, 2.0 wt%, 2.5 wt%, 2.7 wt% ~ 3.0 wt%, 3.2 wt%, 3.5 wt%, 4.0 wt%, 5.0 wt%, 7 wt%, 8.0 wt%, 10.0 wt%, 12 wt%, 15 wt%, 20 wt%, 25 wt%, for example, may be 0.5 wt% ~ 7 wt%, or 1.0 wt% ~ 5 wt%, preferably 2.5 wt% ~ 3.5 wt%, or about 3 wt%. The disintegrant used in the present application is, for example, commercially available croscarmellose sodium SD711 (available from DuPont Nutrition USA).

A lubricant refers to an auxiliary material used to reduce friction between particles to improve powder flowability, which can reduce the differences of weight and the content of active substance between preparations. The lubricants commonly used in the pharmaceutical industry are magnesium stearate and talc, but the inventors of the present application have found that both are auxiliary materials with the aforementioned compatibility problems, thus the pharmaceutical compositions of the present invention are needed to use other lubricants, such as sodium stearic fumarate. In the present application, the lubricant may be added internally or externally. The "added internally" refers to be added when preparing the particles to make the ingredients content between the particles uniform; the "added externally" refers to be added between the prepared particles to make the number of particles contained per unit of the preparation uniform. Unless otherwise stated, the lubricant content described herein refers to the total content of lubricants added internally and externally. The content percentage of the lubricant in the pharmaceutical composition may be, for example, 0.01 wt% ~ 5.0 wt%, such as 0.01 wt%, 0.1 wt%, 0.2 wt%, 0.3 wt%, 0.5 wt% ~ 1.0 wt%, 1.5 wt%, 2.0 wt%, 2.5 wt%, 3.0 wt%, 4.0 wt%, 5.0 wt%, for example, may be 0.1 wt% ~ 2.0 wt%, or 0.2 wt% ~ 1.5 wt%, preferably 0.5 wt% ~ 1.0 wt%, or about 1 wt%. The lubricant used in the present application is, for example, commercially available sodium stearic fumarate (available from Moehs Cantabra).

In addition, the pharmaceutical composition of the present invention may contain other types of auxiliary materials as needed, as long as they do not belong to the aforementioned auxiliary materials with compatibility problems. The other types of auxiliary materials are, for example, adhesives, colorants, flavoring agents, wetting agents, preservatives, solubilizers, and the like.

The present invention also relates to a pharmaceutical preparation containing a pharmaceutical composition as described herein, wherein the pharmaceutical preparation is a solid pharmaceutical preparation known in the art, such as tablets, capsules, granules, powders, pills, and the like. The pharmaceutical preparation may also contain other ingredients known in the art in addition to the pharmaceutical composition. For example, in the case of tablets, the preparation may also have a coating in addition to the pharmaceutical composition; in the case of capsules, the preparation may also have a capsule shell in addition to the pharmaceutical composition. The aforementioned coating or capsule shell may be coatings (e.g., film coatings, controlled release coatings) or capsule shells (e.g., gelatin capsule shells) well known in the art respectively, and can be obtained using methods well known in the art.

Both the pharmaceutical composition and the pharmaceutical preparation described in the present invention can be prepared using conventional methods in the art. For example, in the case of granules, they may be prepared using dry granulation, wet granulation and other methods known in the art; the granules can be further prepared into tablets by tablet pressing, or filled into capsule shells to prepare into capsules, etc.

The "halogen" and "halo" described in the present invention include fluorine, chlorine, bromine, and iodine.

As used in the context of this specification, the term "about" refers to a range of 10% floating up or down the corresponding numerical value. For example, if the concentration of a certain ingredient is about 5 mM, it means that its concentration is 4.5 to 5.5 mM; if the concentration of a certain ingredient is about 5~10 mM, it means that its concentration is 4.5~11 mM. Unless otherwise stated, the percentages used herein are weight percentages (wt%), generally expressed as based on the total weight of a pharmaceutical composition or preparation.

In some preferred embodiments, the solid form (in particular the D crystal form) of the sodium salt of the TJ0113 compound is prepared by the following methods: obtaining the TJ0113 compound (for example, obtained by the method described in prior art CN115894404A), then dissolving the TJ0113 compound in a reaction medium, and adding a base containing sodium to react to obtain.

In some preferred embodiments, the reaction medium is selected from at least one of methanol, ethanol, isopropanol, tert-butanol, acetone, acetonitrile and ethyl acetate, or a mixture of at least one of methanol, ethanol, isopropanol, tert-butanol, acetone, acetonitrile and ethyl acetate and water, for example, acetone, a mixture of acetone and water or a mixture of acetonitrile and water.

In some preferred embodiments, the base containing sodium is selected from at least one of sodium bicarbonate, sodium carbonate, sodium hydroxide, sodium acetate, sodium formate, sodium methoxide, sodium ethoxide and sodium tert-butoxide.

In some preferred embodiments, the molar ratio of the TJ0113 compound to the base containing sodium in the reaction system is 1: (0.9-1.1).

In some preferred embodiments, dissolving the TJ0113 compound in acetone at 35-55°C (preferably 40-50°C), and adding the sodium bicarbonate solution, and after the solid precipitates, keeping warm and standing for at least 20 minutes (preferably at least 30 minutes), and then cooling to room temperature to obtain a solid form of the sodium salt of the TJ0113 compound.

In some preferred embodiments, dissolving the TJ0113 compound in acetone at 35-55°C, cooling to room temperature and adding sodium bicarbonate solution and stirring, and after the solid precipitates, keeping warm and standing for at least 20 minutes (preferably at least 30 minutes) to obtain a solid form of the sodium salt of the TJ0113 compound.

In some preferred embodiments, dissolving the TJ0113 compound in acetone at 35-55°C, adding sodium bicarbonate solution and stirring, and after the solid precipitates, keeping warm and stirring for at least 20 minutes (preferably at least 30 minutes), and then cooling to room temperature to obtain a solid form of the sodium salt of the TJ0113 compound.

In some preferred embodiments, dissolving the TJ0113 compound in acetone at 35-55°C, adding sodium methoxide methanol solution and stirring, and after the solid precipitates, keeping warm and standing for at least 50 minutes (preferably at least 60 minutes), and then cooling to room temperature and stirring for at least 50 minutes (preferably at least 60 minutes), to obtain a solid form of the sodium salt of the TJ0113 compound.

In some preferred embodiments, dissolving the TJ0113 compound in acetone at 35-55°C, adding sodium bicarbonate solution and stirring, and after the solid precipitates, keeping warm and standing for at least 50 minutes (preferably at least 60 minutes), and after the solid precipitates, keeping warm and standing for at least 50 minutes (preferably at least 60 minutes), to obtain a solid form of the sodium salt of the TJ0113 compound.

Examples of the preparation method of the D crystal form are given in the following preparation examples, and the TJ0113 compounds are all obtained by the method described in the prior art CN115894404A.

### PREPARATION EXAMPLE

### Preparation Example 1

Weighing 0.100 g of TJ0113 compound and adding 4 ml of acetone. Heating to about 45°C, stirring and dissolving completely. Adding 0.223 g of sodium methoxide methanol solution dropwise at about 45°C, there is a small amount of solid when dropping in, and dissolved after stirring. After stirring, a solid precipitates slowly. Keeping warm at about 45°C for 1h. After keeping warm, cooling to about 20°C and stirring for 1h. Leaching and rinsing with a small amount of acetone. Drying under vacuum for about 20h at room temperature (not heated). It is determined as D crystal form by the following characterization method.

| Material name | Addition quantity | Molecular weight | Source (batch number) |
|---|---|---|---|
| TJ0113 | 0.100 | 484.34 | E020388-072-12 |
| 5% sodium methoxide methanol solution | 0.223 | 54 | self-prepared |
| acetone | 4ml | N/A | 5055R210601M |

### Preparation Example 2

Weighing 3.00 g of TJ0113 compound and adding 120 ml of acetone. Heating to about 45°C, stirring and dissolving completely. Adding 5.20 g of 10% sodium bicarbonate aqueous solution dropwise at about 45°C, there is a small amount of solid when dropping in, and dissolved after stirring. After stirring, a solid precipitates slowly. Keeping warm at about 45°C for 1h. After keeping warm, cooling to about 20°C and stirring for 1h. Leaching and rinsing with a small amount of acetone. Drying under vacuum for about 20h at room temperature (not heated). It was determined as D crystal form by the following characterization method.

| Material name | Addition quantity | Molecular weight | Source (batch number) |
|---|---|---|---|
| TJ0113 | 3.00 | 484.34 | E020388-103-15 |
| 10% sodium bicarbonate aqueous solution | 5.20 | 84 | self-prepared |
| acetone | 120ml | N/A | 5055R220201M |

### Characterization

X-ray diffraction: The D crystal form prepared above was analyzed using a X-ray powder diffraction analyzer PANalytacal Empyrean. Scan parameters are as follows.

| Parameters | XRPD (reflection mode) |
|---|---|
| X-ray | Cu, kα, Kα1 (Å): λ=1.540598; Kα2 (Å): λ=1.544426 |
| | Kα2/Kα1 Intensity ratio:0.50 |
| X-ray tube setting | 30 kV,10 mA |
| Emission slit | 1.0mm |
| Receiving slit | 3mm |
| Scanning mode | continuous |
| Scanning range (°2Theta) | 4°~40°(2θ) |
| Scanning step (°2Theta) | 0.02°(2θ) |
| Scanning rate (s/step) | 0.2 s/step |

The X-ray powder diffraction patterns of the D crystal forms prepared by Preparation Examples 1 and 2 are shown in Fig. 8, wherein the peak position and intensity of the characteristic peaks are given as follows.

| Angle | d Value | Rel. Intensity |
|---|---|---|
| 34.481 ° | 2.59903 Å | 1.9% |
| 39.178 ° | 2.29754 Å | 2.2% |
| 13.592 ° | 6.50929 Å | 2.9% |
| 31.870 ° | 2.80574 Å | 3.2% |
| 12321 ° | 7.17776 Å | 3.4% |
| 34.678 ° | 2.58470 Å | 3.8% |
| 30.448 ° | 2.93348 Å | 4.1% |
| 28.287 ° | 3.15246 Å | 4.2% |
| 36.676 ° | 2.44834 Å | 4.4% |
| 12.555 ° | 7.04456 Å | 4.9% |
| 31.371 ° | 2.84918 Å | 4.9% |
| 35.305 ° | 2.54019 Å | 5.3% |
| 32.470 ° | 2.75521 Å | 6.2% |
| 18.807 ° | 4.71455 Å | 7.9% |
| 33.455 ° | 2.67631 Å | 7.9% |
| 29.893 ° | 2.98662 Å | 8.5% |
| 38.331 ° | 2.34635 Å | 8.8% |
| 25.384 ° | 3.50600 Å | 11.6% |
| 14.083 ° | 6.28381 Å | 12.2% |
| 16.229 ° | 5.45731 Å | 12.9% |
| 22.633 ° | 3.92552 Å | 15.7% |
| 7.066 ° | 12.49949 Å | 17.9% |
| 37.114 ° | 2.42042 Å | 18.9% |
| 25.911 ° | 3.43584 Å | 20.2% |
| 21.679 ° | 4.09613 Å | 21.0% |
| 27.049 ° | 3.29388 Å | 22.99% |
| 27.654 ° | 3.22319 Å | 23.2% |
| 23.910 ° | 3.71862 Å | 26.8% |
| 10.546 ° | 8.38159 Å | 31.8% |
| 17.584 ° | 5.03962 Å | 39.8% |
| 25.019 ° | 3.55635 Å | 49.1% |
| 18.073 ° | 4.90447 Å | 99.6% |
| 20.875 ° | 4.25200 Å | 100.0% |

Differential scanning calorimetry and DSC pattern: The prepared D crystal form was tested using a differential scanning calorimeter TAQ200/2000, the test parameters are as follows:

| Parameters | DSC |
|---|---|
| Method | Heating linearly |
| Sample disc | Aluminum crucible with a cover with holes |
| Temperature range | 30°C-250°C |
| Scanning rate (°C/min) | 10 |
| Protection gas | N₂ |

The DSC pattern of the D crystal form is shown in Fig. 9; the differential scanning calorimetry curve of the D crystal form has an endothermic peak at 183.79°C (±3°C) and an exothermic peak at 210.79°C±3°C.

Thermogravimetric analysis method and TGA pattern: The prepared D crystal form was tested using a thermogravimetric analyzer TAQ500/5000, the test parameters are as follows:

| Parameters | TGA |
|---|---|
| Method | Heating linearly |
| Sample disc | Aluminum crucible with a cover with holes |
| Temperature range | 30°C-250°C |
| Scanning rate (°C/min) | 10 |
| Protection gas | N₂ |

The TGA pattern of the D crystal form is shown in Fig. 10, and the thermogravimetric analysis curve of D crystal form shows that the sample has no weight loss before decomposition.

### EXAMPLE

The technical solutions and embodiments of the present invention will be exemplified by the capsule preparation of compound TJ0113. It should be understood that these examples are not intended to limit the scope of protection of the invention, and in particular not to limit the active ingredients and specific dosage forms. The "active ingredient" or "API" described in the Examples section is TJ0113, which is in the solid form of sodium salt of D crystal form and is prepared according to the aforementioned preparation examples. The product batch number of raw material is: A16569-028P1.

### Example 1 Compatibility test of active ingredients (API) and various auxiliary materials

The experimental steps are as follows:
1. Taking out the sample according to the sampling time point set in the raw material and auxiliary material compatibility scheme (see Table 1).
2. Recording the appearance of the sample taken out. Appearance abnormalities include: (1) caking, (2) liquefaction, (3) discoloration.
3. Detecting the hygroscopic weight gain of the sample.
4. Using HPLC method to detect the content of the sample and relevant substances.

The parameters of the HPLC method for the detection of the content and relevant substances are detailed in Table 2 below.

The commonly used auxiliary materials for oral solid preparations are selected to mix with TJ0113 raw material in different proportions, placed under different conditions, and taken out at different time points respectively to judge the compatibility of the raw and auxiliary materials by using appearance, hygroscopic weight gain, content and relevant substances as indicators. According to the auxiliary material manual, the auxiliary materials used are commonly used auxiliary materials for oral solid preparations, and have been included in the current edition of the Chinese Pharmacopoeia, each auxiliary material has a stable nature. In order to objectively evaluate the compatibility of raw material and auxiliary materials used, the compatibility test was conducted on the raw material and each auxiliary material.

The ratio of the raw and auxiliary materials is set according to the "Basic Technical Guidance Principles for Research on Chemical Drug Preparations" and the preparation specifications, the auxiliary materials with a larger amount (such as lactose, microcrystalline cellulose) are mixed according to the ratio of main drug: diluent = 1:5; the auxiliary materials with a moderate amount (such as croscarmellose sodium) are mixed according to the ratio of main drug: disintegrant = 1:1; the auxiliary materials with a smaller amount (such as magnesium stearate) are mixed according to the ratio of main drug: lubricant = 10:1. The manufacturer information and the ratio of raw and auxiliary materials for the compatibility experiment are shown in Table 1 below:

**Table 1. Main sample information and ratio of raw and auxiliary materials for the compatibility test**

| **Material name** | **Manufacturer** | **Usage** | **Ratio of raw and auxiliary materials (API:auxiliary material)** |
|---|---|---|---|
| TJ0113 | Pharmaron (Ningbo) Technology Development Co., Ltd | API | N/A |
| Microcrystalline cellulose 102 | Microcellulose Weissenborn GmbH + Co. KG | diluent | 1:5 |
| Lactose Tablettose80 | MEGGLE GmbH & Co.KG | diluent | 1:5 |
| Pregelatinized starch STARCH 1500 ^{®} | Colorcon | diluent | 1:5 |
| Mannitol 100SD | Roquette French | diluent | 1:5 |
| Hydroxypropyl cellulose EXF | Ashland Specialty Ingredients G.P. | adhesive | 1:1 |
| Sodium carboxymethyl starch VIVASTAR P | CHP Carbohydrate Pirna GmbH & Co. KG | disintegrant | 1:1 |
| Croscarmellose sodium SD711 | DuPont Nutrition USA | disintegrant | 1:1 |
| Crospovidone XL-10 | ISP Chemicals LLC | disintegrant | 1:1 |
| Colloidal silica AEROSIL^{®} 200 Pharma | Evonik Operations GmbH | glidant | 10:1 |
| Magnesium stearate LIGAMED MF-2-V-MB | Peter Greven Nederland C.V. | lubricant | 10:1 |
| Sodium stearyl fumarate | Moehs Cantabra | lubricant | 10:1 |
| Talc LUZENAC PHARMA | Imerys Talc Italy S.p.A. | lubricant | 10:1 |
| Gelatin hollow capsules, 0# opaque rich yellow (cap color number: 41.813, body color number: 41.813) -CN, US | Suzhou Capsugel Co., Ltd | capsule shell | 10:1 |
| Hydroxypropyl methylcellulose hollow capsules Vcaps ^{®} Plus, 4# opaque Swedish orange (cap color number: v22.902, body color number: v22.902) -CN, US | Capsugel France SAS | capsule shell | 10:1 |
| Film coating premix (stomach soluble) - Opadry ^{®}85F620077-CN,US | Shanghai Colorcon Coating Technology Ltd | coating material | 10:1 |

**Table 2. Chromatographic measurement conditions**

| Chromatographic column | Agilent poroshell 120 SB-Aq 4.6*150 mm, 2.7 µm | | |
|---|---|---|---|
| Column temperature | 40°C | | |
| Sampling volume | 5 µL | | |
| Flow rate | 1.0 mL/min | | |
| Detection wavelength | 235 nm | | |
| Sample disc temperature | 5°C | | |
| Mobile phase | A:0.2%TFA in water | | |
| | B:0.2%TFA in acetonitrile | | |
| Run time | 48.0 min | | |
| Gradient table | Time(min) | % Mobile phase A | % Mobile phase B |
| | 0.0 | 90 | 10 |
| | 14.0 | 70 | 30 |
| | 30.0 | 55 | 45 |
| | 38.0 | 10 | 90 |
| | 43.0 | 10 | 90 |
| | 43.5 | 90 | 10 |
| | 48.0 | 90 | 10 |
| Diluent | 0.05% TFA in (ACN:H₂O=90:10) | | |
| Sample concentration | 0.4 mg/mL | | |

Placing the samples under experimental conditions of high temperature (60°C), high humidity (92.5% RH), acceleration (40°C/75% RH) and illumination (visible light 5500Lux±500Lux, near ultraviolet light 85µw/cm2), and sampling and investigating on day 0, day 9 and day 30. Submitting the samples for relevant indicator testing (test items include appearance, hygroscopic weight gain, content and relevant substances), and comparing the test results with the control samples (raw material). Relevant results are shown in Tables 3-9 below.

**Table 3. Results of Appearance Test**

| Batch No. | Sample | Initial | 60°C (opened) | | 92.5%RH (opened) | | 40°C/75%R H (opened) | | Illumination( opened) |
|---|---|---|---|---|---|---|---|---|---|
| | | | 10d | 30d | 10d | 30d | 10d | 30d | 10d |
| 1 | API | Light pink powder | Light pink powder | Light pink powder | Light pink powder | Gray powder | Gray pow der | Gray pow der | Powder, the surface is red and the lower layer is light pink |
| 2 | API:microcrystal line cellulose 102 | Off white powder | Off white powder | Off white powder | Off white powder | Off white powder | Off white powder | Gray powder | Powder, the surface is red and the lower layer is off white |
| 3 | API:lactose Tablettose80 | Off white powder | Off white powder | Off white powder | Off white powder | Off white powder | Off white powder | Gray powder | Powder, the surface is red and the lower layer is off white |
| 4 | API:pregelatinized STARCH 1500 ^{®} | Off white powder | Off white powder | Off white powder | Off white, slightly caking | Off white, slightly caking | Slight gray powder | Gray powder | Powder, the surface is red and the lower layer is off white |
| 5 | API:mannitol 100SD | Off white powder | Off white powder | Off white powder | Off white powder | Off white powder | Off white powder | Gray powder | Powder, the surface is red and the lower layer is off white |
| 6 | API:hydroxypro pyl cellulose EXF | Light pink powder | Light pink powder | Light pink powder | Red, caking | Red, caking | Light pink powder | Gray powder | Powder, the surface is red and the lower layer is light pink |
| 7 | API:sodium carboxymethyl starch VIVASTAR P | Light pink powder | Light pink powder | Light pink powder | Light pink powder | Light pink, caking | Slight gray powder | Gray powder | Powder, the surface is red and the lower layer is light pink |
| 8 | API:croscarmellose sodium SD711 | Light pink powder | Light pink powder | Light pink powder | Light pink powder | Light pink powder | Slight gray powder | Gray powder | Powder, the surface is red and the lower layer is light pink |
| 9 | API:crospovidone XL-10 | Light pink powder | Light pink powder | Light pink powder | Red, caking | Red, caking | Light pink powder | Red powder | Powder, the surface is red and the lower layer is light pink |
| 10 | API:colloidal silica AEROSIL^{®} 200 Pharma | Light pink powder | Light pink powder | Light pink powder | Light pink powder | Gray powder | Slight gray powder | Gray powder | Powder, the surface is red and the lower layer is light pink |
| 11 | API:magnesium stearate LIGAMED MF-2-V-MB | Light pink powder | Light pink powder | Light pink powder | Light pink powder | Gray powder | Slight gray powder | Gray powder | Powder, the surface is red and the lower layer is light pink |
| 12 | API:sodium stearyl fumarate | Light pink powder | Light pink powder | Light pink powder | Light pink powder | Gray powder | Slight gray powder | Gray powder | Powder, the surface is red and the lower layer is light pink |
| 13 | API:talc LUZENAC PHARMA | Light pink powder | Light pink powder | Light pink powder | Light pink powder | Gray powder | Slight gray powder | Gray powder | Powder, the surface is red and the lower layer is light pink |
| 14 | API:gelatin hollow capsules, 0# opaque rich yellow (cap color number: 41.813, body color number: 41.813) -CN, US | Light pink powder | Light pink powder | Light pink powder | Light pink powder | Gray powder | Slight gray powder | Gray powder | Powder, the surface is red and the lower layer is light pink |

**Table 4. Results of Hygroscopic Weight Gain Test**

| Batch No. | Sample | 92.5%RH (opened) | | 40°C/75%RH (opened) | |
|---|---|---|---|---|---|
| | | 10d | 30d | 10d | 30d |
| 1 | API | 5.5 | 7.3 | -3.6 | -4.2 |
| 2 | API:microcrystalline cellulose 102 | 6.5 | 7.0 | 1.2 | 1.0 |
| 3 | API:lactose Tablettose80 | 0.4 | 0.8 | -0.05 | -0.2 |
| 4 | API:pregelatinized STARCH 1500 ^{®} | 12.3 | 12.3 | 2.0 | 1.8 |
| 5 | API:mannitol 100SD | 1.7 | 2.1 | -0.1 | -0.1 |
| 6 | API:hydroxypropyl cellulose EXF | 11.3 | 12.2 | 0.1 | -0.4 |
| 7 | API:sodium carboxymethyl starch VIVASTAR P | 33.4 | 35.0 | 5.4 | 3.9 |
| 8 | API:croscarmellose sodium SD711 | 26.5 | 28.5 | 3.1 | 2.6 |
| 9 | API:crospovidone XL-10 | 19.5 | 20.7 | 2.7 | 2.7 |
| 10 | API:colloidal silica AEROSIL^{®} 200 Pharma | 3.2 | 4.6 | -1.3 | -0.9 |
| 11 | API:magnesium stearate LIGAMED MF-2-V-MB | 3.9 | 5.4 | -1.7 | -1.4 |
| 12 | API:sodium stearyl fumarate | 2.3 | 3.3 | 1.0 | 1.2 |
| 13 | API:talc LUZENAC PHARMA | 2.7 | 4.0 | -1.1 | -1.3 |
| 14 | API:gelatin hollow capsules, 0# opaque rich yellow (cap color number: 41.813, body color number: 41.813) -CN, US | 5.4 | 5.8 | 0 | -0.2 |

**Table 5. Results of Content Test**

| Batch No. | Sample | Initial | 60°C (opened) | | 92.5%RH (opened) | | 40°C/75% RH (opened) | | Illumination (opened) |
|---|---|---|---|---|---|---|---|---|---|
| | | | 10d | 30d | 10d | 30d | 10d | 30d | 10d |
| 1 | API | 99.7 | 100.1 | 101.4 | 97.8 | 99.9 | 98.9 | 97.3 | 100.8 |
| 2 | API:microcrystalline cellulose 102 | 97.3 | N/A* | 101.4 | N/A | 100.7 | N/A | 97.0 | 99.1 |
| 3 | API:lactose Tablettose80 | 99.1 | N/A | 96.0 | N/A | 96.5 | 93.3 | 93.6 | 94.7 |
| 4 | API:pregelatinized STARCH 1500 ^{®} | 97.9 | N/A | 100.5 | N/A | 99.0 | N/A | 96.3 | 98.9 |
| 5 | API:mannitol 100SD | 98.8 | 99.2 | 97.6 | 97.2 | 97.0 | 98.3 | 95.6 | 96.9 |
| 6 | API:hydroxypropyl cellulose EXF | 97.5 | N/A | 97.7 | 96.5 | 97.4 | 95.0 | 94.2 | 97.8 |
| 7 | API:sodium carboxymethyl starch VIVASTAR P | 95.7 | 96.9 | 97.2 | N/A | 97.3 | 96.6 | 96.0 | 96.0 |
| 8 | API:croscarmellose sodium SD711 | 96.9 | N/A | 97.7 | N/A | 97.1 | N/A | 95.4 | 96.4 |
| 9 | API:crospovidone XL-10 | 96.5 | 97.3 | 95.9 | 96.6 | 91.7 | 92.5 | 95.5 | 98.2 |
| 10 | API:colloidal silica AEROSIL^{®} 200 Pharma | 100.2 | N/A | 100.8 | 96.0 | 99.2 | 101.0 | 94.1 | 99.6 |
| 11 | API:magnesium stearate LIGAMED MF-2-V-MB | 100.3 | N/A | 100.4 | 98.9 | 95.9 | 97.0 | 94.7 | 98.5 |
| 12 | API:sodium stearyl fumarate | 100.2 | N/A | 100.8 | 97.6 | 98.3 | 99.7 | 98.7 | 99.3 |
| 13 | API:talc LUZENAC PHARMA | 100.5 | N/A | 99.2 | 95.0 | 97.8 | 98.5 | 98.9 | 99.0 |
| 14 | API:gelatin hollow capsules, 0# opaque rich yellow (cap color number: 41.813, body color number: 41.813) -CN, US | 99.9 | N/A | 98.9 | N/A | 96.9 | N/A | 94.9 | 99.1 |

| | | | | | | | | | |
|---|---|---|---|---|---|---|---|---|---|
| *N/A: Since the results of the relevant substances did not change significantly in 30 days, the samples were not detected on the day 10 (10d). The results of relevant substance detection are as follows. | | | | | | | | | |

**Table 6. Results of Relevant Substances at 60 °C (opened)**

| Batch No. | API:auxiliary material (ratio) | Days | %Impurities (60 °C, opened) | | | | | | | | | | |
|---|---|---|---|---|---|---|---|---|---|---|---|---|---|
| | | | RRT0.46 | RRT0.89 | RRT1.16 | RRT1.20 | RRT1.29 | RRT1.33 | RRT1.37 | RRT1.41 | RRT1.73 | RRT1.74 | Total |
| 1 | API | 0d | < 0.05 | 0.08 | 0.88 | 0.09 | 0.14 | 0.10 | 0.07 | < 0.05 | < 0.05 | 0.05 | 1.41 |
| | | 10d | < 0.05 | 0.08 | 0.87 | 0.07 | 0.17 | 0.08 | 0.06 | < 0.05 | < 0.05 | 0.07 | 1.40 |
| | | 30d | < 0.05 | 0.07 | 0.87 | 0.06 | 0.18 | 0.07 | 0.06 | < 0.05 | < 0.05 | 0.09 | 1.40 |
| 2 | API:microcrystalline cellulose 102 (1:5) | 0d | < 0.05 | < 0.05 | 0.85 | 0.10 | 0.10 | 0.11 | < 0.05 | < 0.05 | < 0.05 | 0.06 | 1.22 |
| | | 30d | 0.07 | 0.06 | 0.87 | 0.07 | 0.18 | 0.07 | 0.06 | < 0.05 | < 0.05 | 0.09 | 1.47 |
| 3 | API:lactose Tablettose80 (1:5) | 0d | < 0.05 | < 0.05 | 0.86 | 0.11 | 0.12 | 0.11 | 0.06 | < 0.05 | < 0.05 | 0.06 | 1.32 |
| | | 30d | 0.05 | 0.06 | 0.87 | 0.07 | 0.18 | 0.07 | 0.05 | < 0.05 | < 0.05 | 0.08 | 1.43 |
| 4 | API:pregelatinized STARCH 1500 ^{®} (1:5) | 0d | < 0.05 | < 0.05 | 0.84 | 0.09 | 0.13 | 0.11 | < 0.05 | < 0.05 | < 0.05 | 0.06 | 1.23 |
| | | 30d | 0.07 | 0.05 | 0.88 | 0.07 | 0.20 | 0.07 | 0.05 | < 0.05 | < 0.05 | 0.09 | 1.48 |
| 5 | API:mannitol 100SD (1:5) | 0d | < 0.05 | 0.06 | 0.86 | 0.10 | 0.13 | 0.11 | < 0.05 | < 0.05 | < 0.05 | 0.06 | 1.32 |
| | | 10d | < 0.05 | 0.06 | 0.88 | 0.08 | 0.17 | 0.08 | 0.06 | < 0.05 | 0.09 | 0.07 | 1.49 |
| | | 30d | 0.07 | 0.07 | 0.88 | 0.06 | 0.23 | 0.07 | 0.06 | 0.06 | 0.10 | 0.08 | 1.68 |
| 6 | API:hydroxypropyl cellulose EXF (1:1) | 0d | < 0.05 | 0.07 | 0.86 | 0.10 | 0.12 | 0.10 | 0.05 | < 0.05 | < 0.05 | 0.05 | 1.35 |
| | | 30d | 0.07 | 0.05 | 0.88 | 0.07 | 0.20 | 0.07 | < 0.05 | < 0.05 | < 0.05 | 0.08 | 1.42 |
| 7 | API:sodium carboxymethyl starch VIVASTAR P (1:1) | 0d | < 0.05 | < 0.05 | 0.83 | 0.10 | 0.17 | 0.11 | < 0.05 | < 0.05 | < 0.05 | 0.06 | 1.27 |
| | | 10d | < 0.05 | < 0.05 | 0.88 | 0.08 | 0.21 | 0.08 | 0.05 | < 0.05 | < 0.05 | 0.07 | 1.37 |
| | | 30d | 0.05 | < 0.05 | 0.86 | 0.06 | 0.23 | 0.07 | 0.05 | < 0.05 | < 0.05 | 0.09 | 1.41 |
| 8 | API:croscarmellose sodium SD711 (1:1) | 0d | < 0.05 | < 0.05 | 0.85 | 0.10 | 0.14 | 0.10 | < 0.05 | < 0.05 | < 0.05 | 0.06 | 1.25 |
| | | 30d | 0.05 | < 0.05 | 0.86 | 0.06 | 0.20 | 0.07 | 0.05 | < 0.05 | < 0.05 | 0.09 | 1.38 |
| 9 | API:crospovidone XL-10 (1:1) | 0d | < 0.05 | < 0.05 | 0.84 | 0.10 | 0.16 | 0.10 | 0.06 | < 0.05 | ND | 0.06 | 1.32 |
| | | 10d | 0.05 | < 0.05 | 0.87 | 0.08 | 0.19 | 0.08 | 0.06 | < 0.05 | < 0.05 | 0.08 | 1.41 |
| | | 30d | 0.08 | 0.05 | 0.91 | 0.06 | 0.19 | 0.07 | 0.09 | < 0.05 | < 0.05 | 0.10 | 1.55 |
| 10 | API:colloidal silica AEROSIL^{®} 200 Pharma (10:1) | 0d | < 0.05 | 0.08 | 0.87 | 0.10 | 0.13 | 0.11 | < 0.05 | < 0.05 | < 0.05 | 0.06 | 1.35 |
| | | 30d | 0.07 | 0.06 | 0.87 | 0.06 | 0.17 | 0.07 | 0.05 | < 0.05 | 0.06 | 0.09 | 1.50 |
| 11 | API:magnesium stearate LIGAMED MF-2-V-MB (10:1) | 0d | < 0.05 | 0.06 | 0.85 | 0.10 | 0.16 | 0.10 | 0.05 | < 0.05 | < 0.05 | 0.06 | 1.38 |
| | | 30d | 0.06 | 0.07 | 0.87 | 0.07 | 0.18 | 0.07 | 0.05 | < 0.05 | < 0.05 | 0.10 | 1.47 |
| 12 | API:sodium stearyl fumarate (10:1) | 0d | < 0.05 | 0.06 | 0.85 | 0.10 | 0.14 | 0.11 | < 0.05 | < 0.05 | < 0.05 | 0.06 | 1.32 |
| | | 30d | 0.05 | 0.07 | 0.87 | 0.06 | 0.17 | 0.06 | 0.05 | < 0.05 | < 0.05 | 0.10 | 1.43 |
| 13 | API:talc LUZENAC PHARMA (10:1) | 0d | < 0.05 | 0.08 | 0.88 | 0.09 | 0.11 | 0.11 | 0.06 | < 0.05 | < 0.05 | 0.06 | 1.39 |
| | | 30d | 0.05 | 0.10 | 0.89 | 0.06 | 0.16 | 0.07 | 0.09 | < 0.05 | 0.05 | 0.10 | 1.57 |
| 14 | API:gelatin hollow capsules, 0# (10:1) | 0d | < 0.05 | < 0.05 | 0.85 | 0.10 | 0.15 | 0.10 | < 0.05 | < 0.05 | < 0.05 | 0.06 | 1.26 |
| | | 30d | 0.05 | 0.05 | 0.87 | 0.06 | 0.19 | 0.07 | < 0.05 | < 0.05 | < 0.05 | 0.10 | 1.39 |

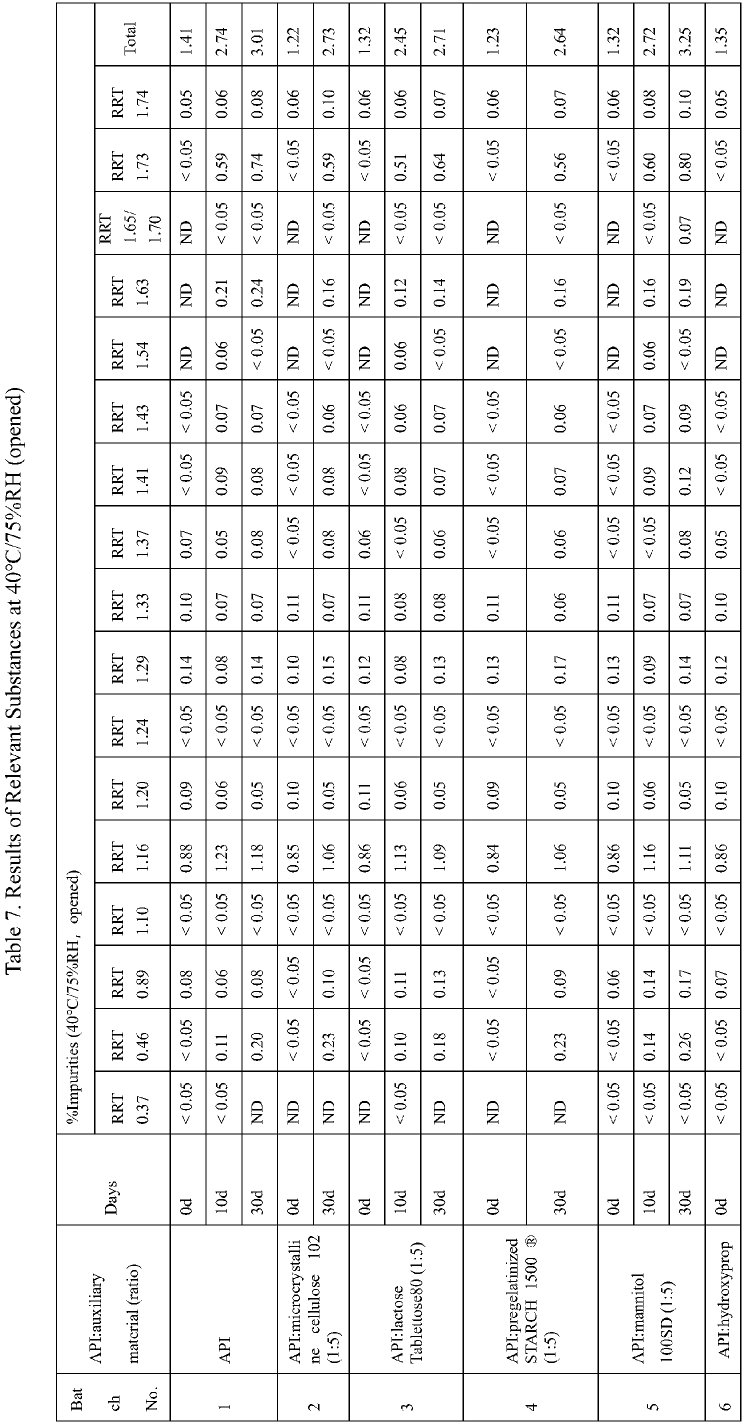

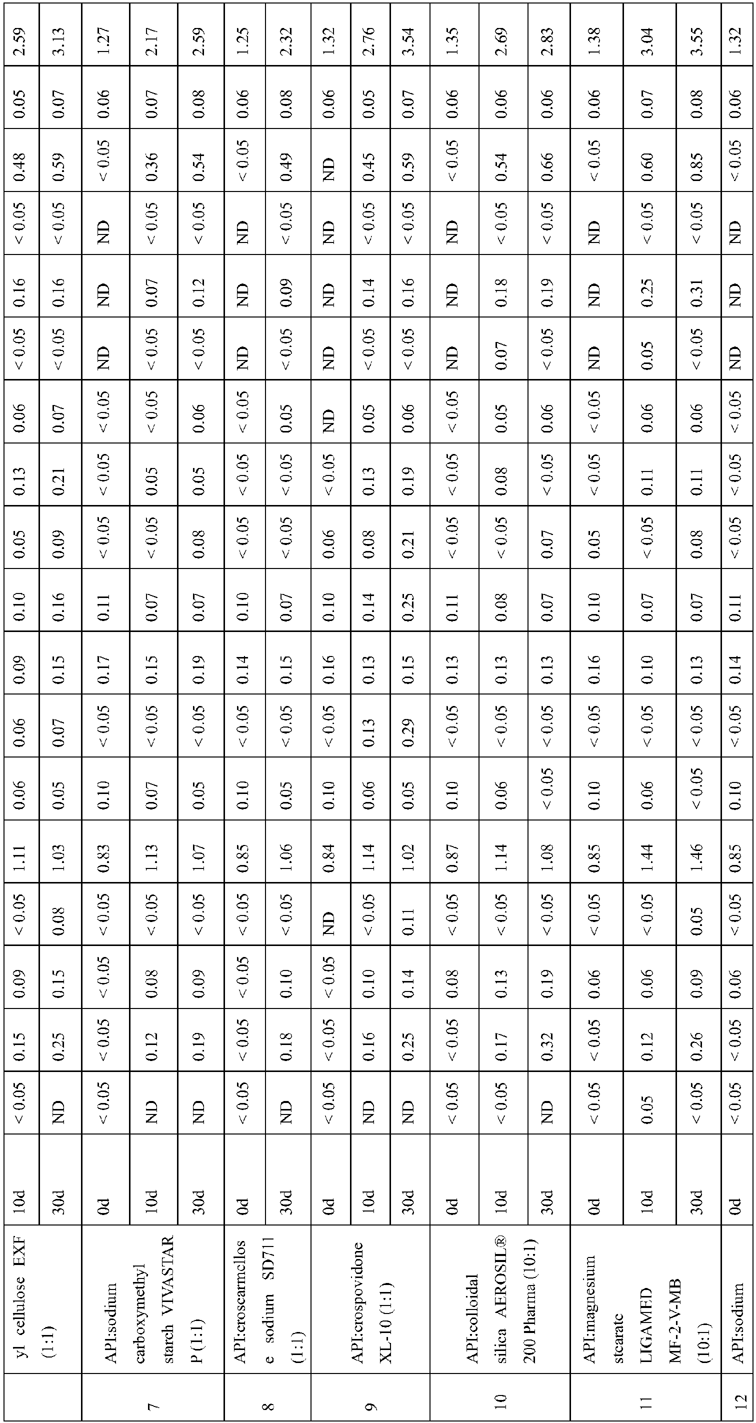

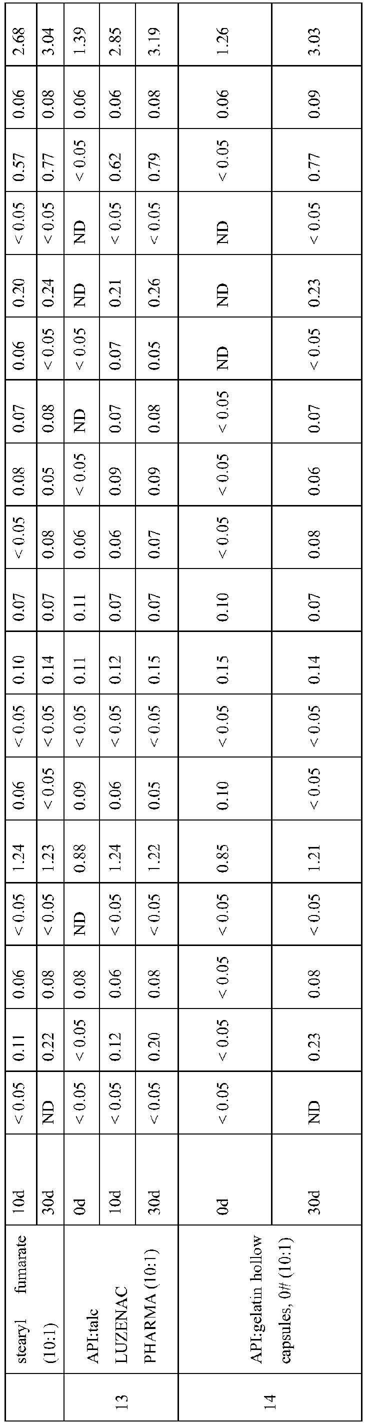

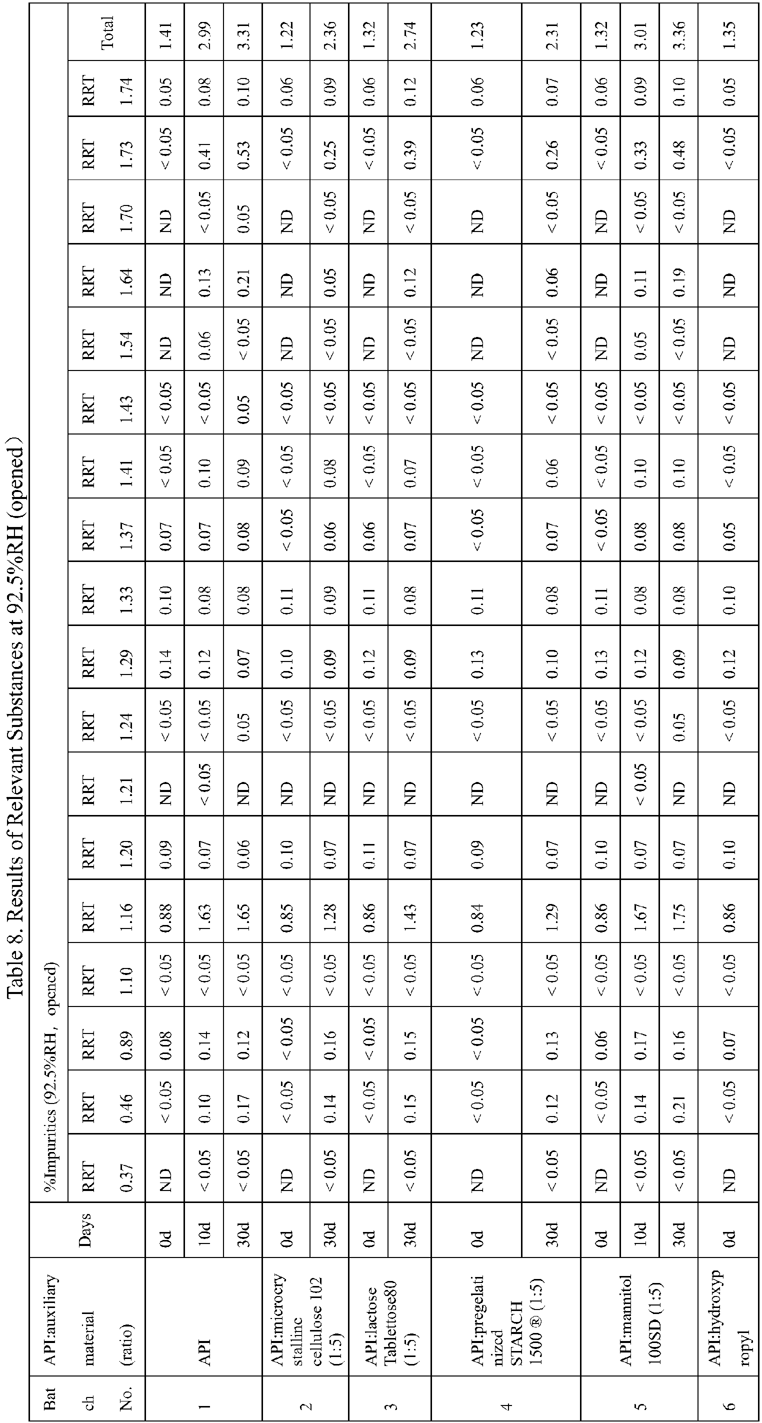

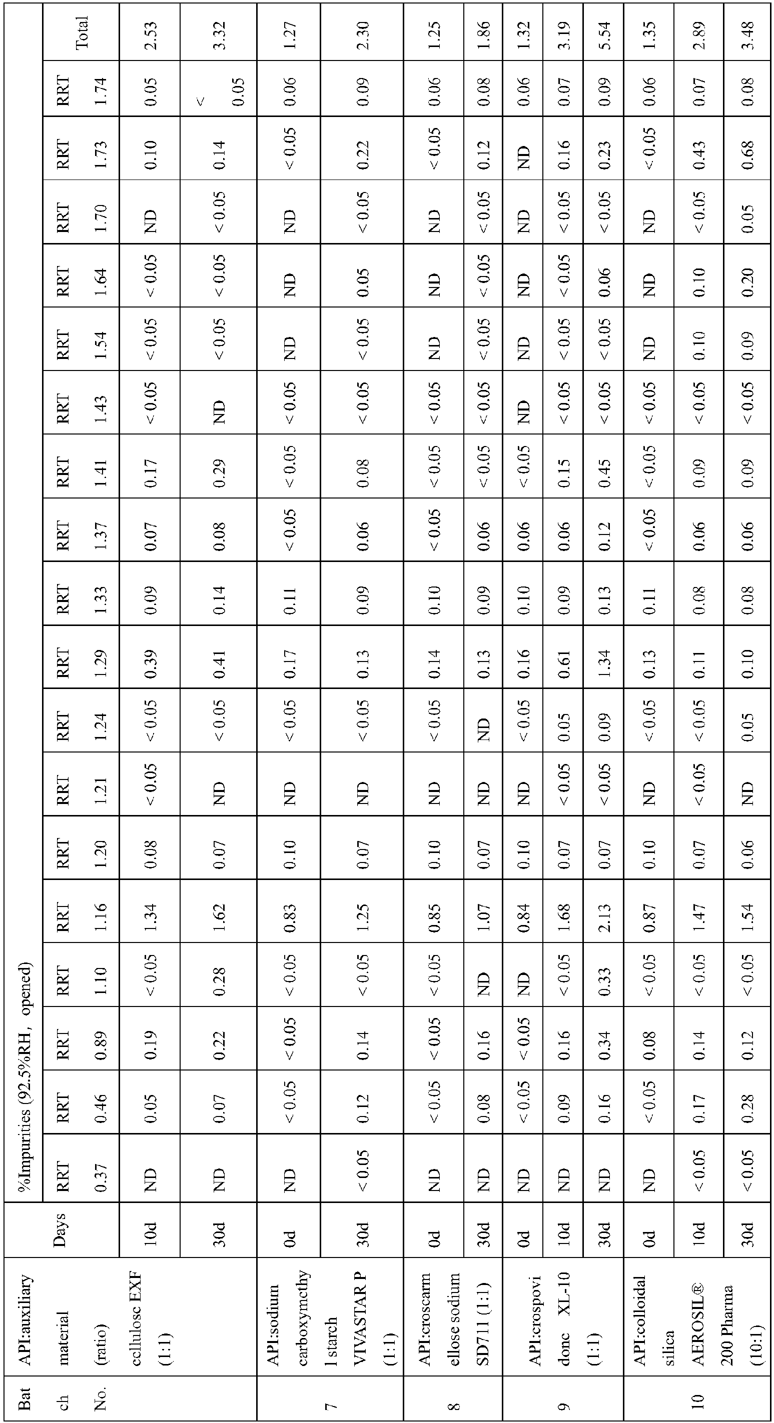

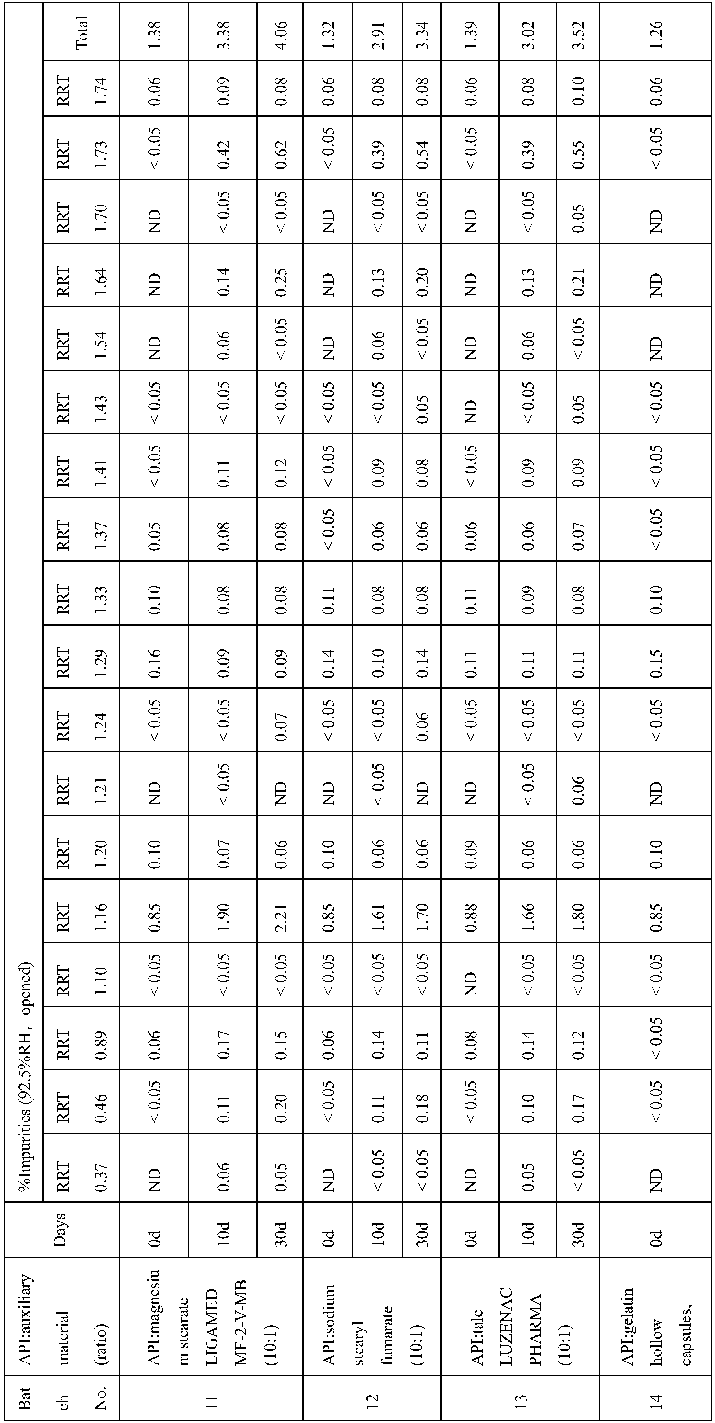

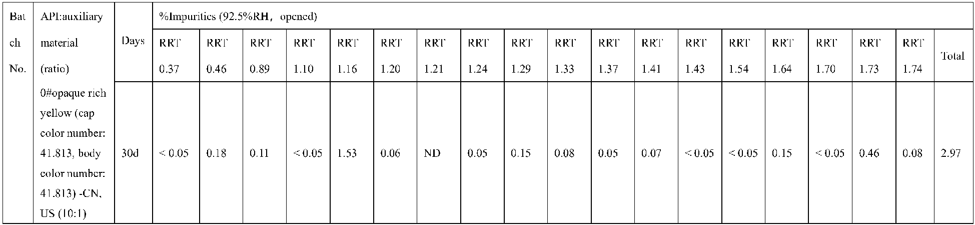

**Table 8. Results of Relevant Substances under Illumination (opened)**

| Batch No. | API:auxiliary material (ratio) | Days | %Impurities (Illumination, opened) | | | | | | | | | | |
|---|---|---|---|---|---|---|---|---|---|---|---|---|---|
| | | | RRT0.46 | RRT0.89 | RRT1.16 | RRT1.20 | RRT1.29 | RRT1.33 | RRT1.37 | RRT1.41 | RRT1.73 | RRT1.74 | Total |
| 1 | API | 0d | < 0.05 | 0.08 | 0.88 | 0.09 | 0.14 | 0.10 | 0.07 | < 0.05 | < 0.05 | 0.05 | 1.41 |
| | | 10d | 0.06 | 0.17 | 0.91 | 0.08 | 0.20 | 0.09 | 0.07 | < 0.05 | 0.05 | 0.07 | 1.70 |
| 2 | API: microcrystalline cellulose 102 (1:5) | 0d | < 0.05 | < 0.05 | 0.85 | 0.10 | 0.10 | 0.11 | < 0.05 | < 0.05 | < 0.05 | 0.06 | 1.22 |
| | | 10d | 0.08 | 0.15 | 0.90 | 0.08 | 0.23 | 0.09 | 0.06 | < 0.05 | 0.07 | < 0.05 | 1.66 |
| 3 | API:lactose Tablettose80 (1:5) | 0d | < 0.05 | < 0.05 | 0.86 | 0.11 | 0.12 | 0.11 | 0.06 | < 0.05 | < 0.05 | 0.06 | 1.32 |
| | | 10d | 0.09 | 0.19 | 0.91 | 0.08 | 0.22 | 0.09 | 0.05 | < 0.05 | 0.07 | 0.06 | 1.76 |
| 4 | API:pregelatinized STARCH 1500 ^{®} (1:5) | 0d | < 0.05 | < 0.05 | 0.84 | 0.09 | 0.13 | 0.11 | < 0.05 | < 0.05 | < 0.05 | 0.06 | 1.23 |
| | | 10d | 0.06 | 0.17 | 0.89 | 0.08 | 0.23 | 0.08 | < 0.05 | < 0.05 | 0.05 | 0.06 | 1.62 |
| 5 | API:mannitol 100SD (1:5) | 0d | < 0.05 | 0.06 | 0.86 | 0.10 | 0.13 | 0.11 | < 0.05 | < 0.05 | < 0.05 | 0.06 | 1.32 |
| | | 10d | 0.07 | 0.17 | 0.91 | 0.08 | 0.22 | 0.09 | 0.07 | < 0.05 | 0.07 | 0.07 | 1.75 |
| 6 | API:hydroxypropyl cellulose EXF (1:1) | 0d | < 0.05 | 0.07 | 0.86 | 0.10 | 0.12 | 0.10 | 0.05 | < 0.05 | < 0.05 | 0.05 | 1.35 |
| | | 10d | 0.08 | 0.17 | 0.90 | 0.08 | 0.22 | 0.08 | 0.06 | < 0.05 | < 0.05 | 0.06 | 1.65 |
| 7 | API:sodium carboxymethyl starch VIVASTAR P (1:1) | 0d | < 0.05 | < 0.05 | 0.83 | 0.10 | 0.17 | 0.11 | < 0.05 | < 0.05 | < 0.05 | 0.06 | 1.27 |
| | | 10d | 0.06 | 0.13 | 0.89 | 0.09 | 0.24 | 0.09 | 0.05 | < 0.05 | 0.05 | 0.07 | 1.67 |
| 8 | API:croscarmellose sodium SD711 (1:1) | 0d | < 0.05 | < 0.05 | 0.85 | 0.10 | 0.14 | 0.10 | < 0.05 | < 0.05 | < 0.05 | 0.06 | 1.25 |
| | | 10d | 0.06 | 0.18 | 0.89 | 0.08 | 0.30 | 0.09 | 0.05 | < 0.05 | 0.08 | 0.08 | 1.81 |
| 9 | API:crospovidone XL-10 (1:1) | 0d | < 0.05 | < 0.05 | 0.84 | 0.10 | 0.16 | 0.10 | 0.06 | < 0.05 | ND | 0.06 | 1.32 |
| | | 10d | 0.09 | 0.18 | 0.91 | 0.08 | 0.29 | 0.09 | 0.06 | 0.06 | < 0.05 | 0.06 | 1.82 |
| 10 | API:colloidal silica AEROSIL^{®} 200 Pharma (10:1) | 0d | < 0.05 | 0.08 | 0.87 | 0.10 | 0.13 | 0.11 | < 0.05 | < 0.05 | < 0.05 | 0.06 | 1.35 |
| | | 10d | 0.08 | 0.15 | 0.92 | 0.09 | 0.24 | 0.08 | 0.07 | < 0.05 | 0.08 | 0.07 | 1.78 |
| 11 | API:magnesium stearate LIGAMED MF-2-V-MB (10:1) | 0d | < 0.05 | 0.06 | 0.85 | 0.10 | 0.16 | 0.10 | 0.05 | < 0.05 | < 0.05 | 0.06 | 1.38 |
| | | 10d | 0.07 | 0.16 | 0.91 | 0.08 | 0.22 | 0.09 | 0.07 | < 0.05 | 0.05 | 0.07 | 1.72 |
| 12 | API: sodium stearyl fumarate (10:1) | 0d | < 0.05 | 0.06 | 0.85 | 0.10 | 0.14 | 0.11 | < 0.05 | < 0.05 | < 0.05 | 0.06 | 1.32 |
| | | 10d | 0.07 | 0.21 | 0.91 | 0.08 | 0.29 | 0.09 | 0.07 | < 0.05 | 0.06 | 0.07 | 1.85 |
| 13 | API:talc LUZENAC PHARMA (10:1) | 0d | < 0.05 | 0.08 | 0.88 | 0.09 | 0.11 | 0.11 | 0.06 | < 0.05 | < 0.05 | 0.06 | 1.39 |
| | | 10d | 0.06 | 0.18 | 1.09 | 0.08 | 0.24 | 0.08 | 0.06 | 0.06 | 0.10 | 0.06 | 2.01 |
| 14 | API:gelatin hollow capsules, 0#opaque rich yellow (cap color number: 41.813, body color number: 41.813) -CN, US (10:1) | 0d | < 0.05 | < 0.05 | 0.85 | 0.10 | 0.15 | 0.10 | < 0.05 | < 0.05 | < 0.05 | 0.06 | 1.26 |
| | | 10d | 0.07 | 0.16 | 0.91 | 0.08 | 0.24 | 0.09 | 0.06 | < 0.05 | 0.06 | 0.07 | 1.74 |

The following is a discussion of experimental results on the compatibility of the API with multiple auxiliary materials:

### (1) Appearance:

After opened placement under the condition of acceleration40°C/75%RH for 30 days, except that the mixed sample of API + crospovidone XL-10 turned into red powder, the API and mixed samples of API and other auxiliary materials turned into gray powder.
After opened placement under the condition of high humidity 92.5%RH for 30 days, the API, mixed samples of API + colloidal silica 200Pharma, API + Magnesium Stearate LIGAMED MF-2-V-MB, API + Sodium Stearic Fumarate, API + Talc PHARMA, API + Gelatin Hollow Capsules and 0# opaque rich yellow (Cap Color Number: 41.813, Body Color Number: 41.813)-CN, US turned into gray powder, and the mixed samples of API + hydroxypropyl cellulose EXF and API + crospovidone XL-10 turned into red.

After opened placement under the condition of high humidity 92.5%RH for 30 days, the mixed samples of API + pregelatinized starch STARCH 1500 ^{®}, API + hydroxypropyl cellulose EXF, API + crospovidone XL-10 and API + carboxymethyl starch sodium VIVASTAR P showed caking.

After opened placement under the condition of illumination for 30 days, the surfaces of the API, mixed samples of API and other auxiliary materials turned into red, and their lower layers did not change color.

Apart from these, no significant appearance changes were observed in the mixed samples of API and other auxiliary materials.

### (2) Hygroscopic weight gain:

After opened placement under the condition of high humidity 92.5%RH for 30 days, the API, mixed samples of API + microcrystalline cellulose 102, API + pregelatinized starch STARCH 1500 ^{®}, API + hydroxypropyl cellulose EXF, API + carboxymethyl starch sodium VIVASTARP, API + Croscarmellose Sodium SD711, API + crospovidone XL-10, API + magnesium stearate LIGAMED MF-2-V-MB and API + gelatin hollow capsules 0# opaque rich yellow had larger hygroscopic weight gain.

### (3) Content:

The content results show that after opened placement under the conditions of high temperature 60°C, high humidity 92.5% RH and acceleration 40°C/75% RH, and after 10 days under illumination condition, the contents of API and the mixed samples of API and the respective auxiliary material did not change significantly.

### (4) Relevant substances:

After opened placement under the condition of high temperatures 60°C for 30 days, the relevant substance of the mixed sample of API + mannitol 100SD increased slightly at RRT1.29 and RRT1.73.

The relevant substance of the mixed sample of API + Carboxymethyl starch sodium VIVASTAR P increased slightly at RRT1.29. The relevant substance of the mixed sample of API + cross-povidone XL-10 slightly increased at RRT 0.46.

After opened placement under condition of acceleration 40°C/75% RH for 30 days, the relevant substance of the API increased significantly at RRT0.46, RRT1.16, RRT1.63 and RRT1.73, and increased slightly at RRT1.41 and RRT1.43. The relevant substance of the mixed sample of API + Lactose Tablettose80 increased slightly at RRT 0.89. The relevant substance of the mixed sample of API + mannitol 100SD increased slightly at RRT 0.46, RRT 0.89, RRT 1.65/1.70 and RRT 1.73. The relevant substance of the mixed sample of API + hydroxypropyl cellulose EXF increased slightly at RRT0.46, RRT0.89, RRT1.10, RRT1.24 and RRT1.33, and increased significantly at RRT1.41. The relevant substance of the mixed sample of API + Carboxymethyl starch sodium VIVASTAR P increased slightly at RRT1.29. The relevant substance of the mixed sample of API + crospovidone XL-10 increased slightly at RRT0.46, RRT0.89 and RRT1.10, and increased significantly at RRT1.24, RRT1.33, RRT1.37 and RRT1.41. The relevant substance of the mixed sample of API + colloidal silica AEROSIL^{®} 200 Pharma increased significantly at RRT 0.46 and RRT 0.89. The relevant substance of the mixed sample of API + Magnesium stearate LIGAMED MF-2-V-MB increased slightly at RRT 0.46 and RRT 1.63, and increased significantly at RRT 1.16 and RRT 1.73. The relevant substance of the mixed sample of API + Sodium Stearic fumarate increased slightly at RRT 1.16. The relevant substance of the mixed sample of API + Talc LUZENAC PHARMA increased slightly at RRT1.73.

After opened placement under the condition of high humidity 92.5% RH for 30 days, the relevant substance of the API increased significantly at RRT0.46, RRT1.16, RRT1.64 and RRT1.73, and increased slightly at RRT1.41 and RRT1.74. The relevant substance of the mixed sample of API + mannitol 100SD increased significantly at RRT1.16. The relevant substance of the mixed sample of API + hydroxypropyl cellulose EXF increased significantly at RRT 0.89, RRT 1.10, RRT 1.29 and RRT 1.41, and slightly increased at RRT 1.33. The relevant substance of the mixed sample of API + crospovidone XL-10 increased significantly at RRT0.89, RRT1.10, RRT1.16, RRT1.29 and RRT1.41, and increased slightly at RRT1.33. The relevant substance of the mixed sample of API + colloidal silica AEROSIL^{®} 200 Pharma increased significantly at RRT 0.46 and RRT 1.73 and increased slightly at RRT 1.54. The relevant substance of the mixed sample of API + magnesium stearate LIGAMED MF-2-V-MB increased significantly at RRT1.16 and increased slightly at RRT1.73. The relevant substance of the mixed sample of API + sodium stearic fumarate increased significantly at RRT 1.16. The relevant substance of the mixed sample of API + talc LUZENAC PHARMA increased significantly at RRT1.16 and increased slightly at RRT1.21.

After opened placement under illumination for 30 days, the relevant substance of the API increased slightly at RRT 0.89 and RRT 1.29. The relevant substance of the mixed sample of API + croscarmellose sodium SD711 increased significantly at RRT1.29. The relevant substances of the mixed samples of API + crospovidone XL-10 and API + sodium stearyl fumarate increased slightly at RRT1.29. The relevant substance of the mixed sample of API + talc LUZENACPHARMA increased significantly at RRT1.16 and increased slightly at RRT1.73.

In summary, the results of the compatibility test of raw and auxiliary materials show that API is unstable under the conditions of high humidity, illumination and acceleration, the mixed samples of API with hydroxypropyl cellulose EXF, crospovidone XL-10, colloidal silica AEROSIL^{®} 200 Pharma and magnesium stearate LIGAMED MF-2-V-MB are incompatible under the conditions of acceleration 40°C/75% RH and high humidity 92.5% RH. The mixed sample of API with talc LUZENAC PHARMA is incompatible under the conditions of high humidity 92.5% RH opened and illumination. The mixed samples of API with mannitol 100SD is incompatible under the condition of high humidity 92.5% RH. The mixture sample of API + croscarmellose sodium SD711 is incompatible under illumination condition.

### Example 2: Effect of diluent on dissolution performance

Considering that TJ0113 itself is unstable under high humidity conditions and combined with the compatibility results of raw and auxiliary materials, the influence of diluent types on the preparation process and capsule dissolution was investigated on this basis. The dissolution methods used in this application to measure dissolution are shown in Table 10.

**Table 10. Dissolution method**

| **Dissolution method** | **Dissolution method 1** | **Dissolution method 2** | **Dissolution method 3(final)** |
|---|---|---|---|
| **Dissolution device** | Chinese Pharmacopoeia 2020 edition, 0931, second method: paddle method + basket sinker | Chinese Pharmacopoeia 2020 edition, 0931, first method: basket method | Chinese Pharmacopoeia 2020 edition, 0931, second method: paddle method + basket sinker |
| **Dissolution medium** | pH 4.5 | pH 4.5 | pH 4.5 |
| **Dissolution medium volume** | 900 mL | 900 mL | 900 mL |
| **Dissolution medium temperature** | 37 ± 0.5 °C | 37 ± 0.5 °C | 37 ± 0.5 °C |
| **Rotation speed of stirring paddle** | 50 rpm (60-75 min is the final rotation speed test, and the rotation speed is 250 rpm) | 75 rpm (60-75 min is the final rotation speed test, and the rotation speed is 250 rpm) | 75 rpm (60-75 min is the final rotation speed test, and the rotation speed is 250 rpm) |
| **Sampling time** | 5 min, 15 min, 30 min, 45 min, 60 min, 75 min | 5 min, 15 min, 30 min, 45 min, 60 min, 75 min | 5 min, 15 min, 30 min, 45 min, 60 min, 75 min |

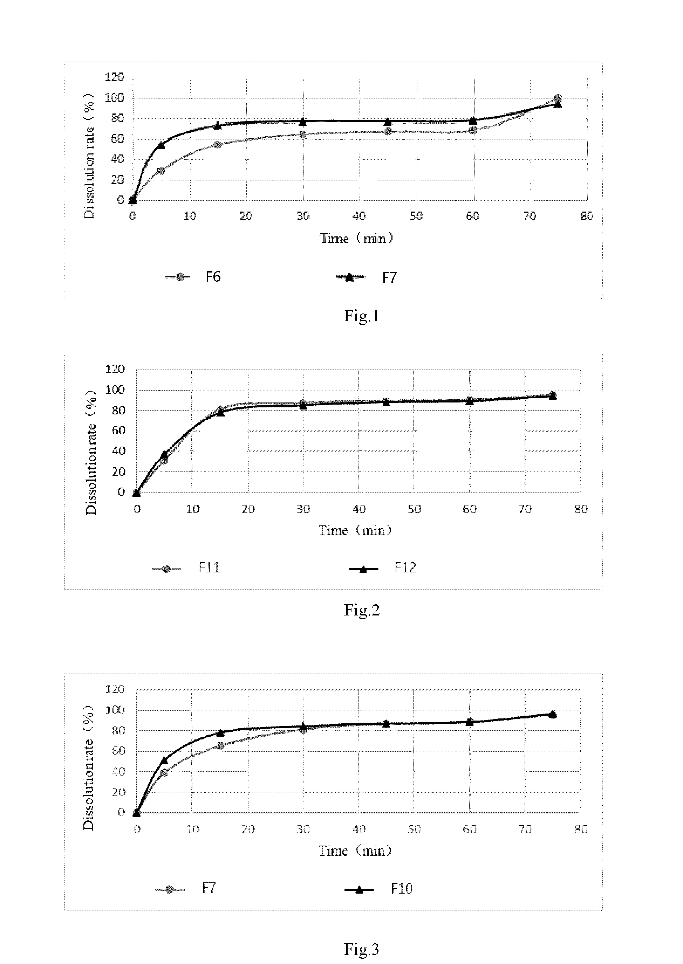

This Example uses 100mg capsules as the main research object, with a total weight of contents of 420mg, 0# opaque rich yellow gelatin hollow capsules are used, and all of the capsules are filled by dry granulation. Table 11 lists the specific ingredients of Experiment NO. F6 and F7, which use different types of diluents. The effect of the diluent type on the dissolution results is shown in Table 12 and Fig. 1, where the dissolution method 1 is used for evaluation.

**Table 11. Specific ingredients of F6 and F7**

| **Experiment No.** | **F6** | | **F7** | |
|---|---|---|---|---|
| **Diluent** | **Anhydrous lactose DT HV** | | **Microcrystalline cellulose 102 and pregelatinized starch STARCH 1500 ^{®}** | |
| **Ingredient** | **Percentage%** | **Unit dosage mg** | **Percentage%** | **Unit dosage mg** |
| Added internally | | | | |
| TJ0113 | 24.952 | 104.80* | 24.952 | 104.80* |
| Anhydrous lactose DT HV | 71.048 | 298.40 | N/A | N/A |
| Microcrystalline cellulose 102 | N/A | N/A | 56.048 | 235.40 |
| pregelatinized starch STARCH 1500 ^{®} | N/A | N/A | 15.000 | 63.00 |
| Croscarmellose Sodium SD711 | 3.000 | 12.60 | 3.000 | 12.60 |
| sodium stearyl fumarate | 0.500 | 2.10 | 0.500 | 2.10 |

| Added externally | | | | |
|---|---|---|---|---|
| sodium stearyl fumarate | 0.500 | 2.10 | 0.500 | 2.10 |
| total | 100.000 | 420.00 | 100.000 | 420.00 |

| Capsule shell | | | | |
|---|---|---|---|---|
| Gelatin hollow capsule, 0# | 1 capsule | 96.00 | 1 capsule | 96.00 |
| Remarks: * the specification is based on free acid radical, the molecular weight of free acid radical is 483.33, TJ0113 is sodium salt, its molecular weight is 506.32, and the conversion coefficient is 1.048. | | | | |

**Table 12. Effects of diluent types on dissolution results (dissolution method 1)**

| **Experiment No.** | **Dissolution item** | **Dissolution rate (%)** | | | | | |
|---|---|---|---|---|---|---|---|
| | | **5 min** | **15 min** | **30 min** | **45 min** | **60 min** | **75 min** |
| F6 | median | 29 | 54 | 64 | 67 | 68 | 99 |
| | RSD% | 26.2 | 20.8 | 14.8 | 13.5 | 12.2 | 0.5 |
| F7 | median | 54 | 73 | 77 | 77 | 78 | 94 |
| | RSD% | 0.3 | 1.5 | 1.5 | 1.2 | 1.2 | 0.4 |

From the dissolution results shown in Fig. 1, it can be seen that the dissolution of F7 is significantly higher than that of F6 (especially within the first 20 minutes), indicating that the combination of microcrystalline cellulose and pregelatinized starch unexpectedly improves the dissolution of the API. Therefore, the diluent types are selected as microcrystalline cellulose 102 and pregelatinized starch STARCH 1500 ^{®}.

Since the relevant substance of the API of the present invention increases significantly under high humidity conditions, and pregelatinized starch STARCH 1500 ^{®} ( 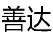 ^{™} ) can absorb free moisture entering the interior of the core and lock in the moisture to avoid interaction with the drug, effect of the pregelatinized starch of different ratios on product dissolution was also investigated. The specific composition is shown in Table 13, and the dissolution results are shown in Table 14 and Fig. 2, where dissolution method 2 is used for evaluation.

**Table 13. Specific ingredients of F11 and F12**

| **Experiment No.** | **F11** | | **F12** | |
|---|---|---|---|---|
| **Weight percentage of pregelatinized starch STARCH 1500 ^{®}** | **25 wt%** | | **15 wt%** | |
| **Ingredient** | **Percentage%** | **Unit dosage mg** | **Percentage%** | **Unit dosage mg** |
| Added internally | | | | |
| TJ0113 | 24.952 | 104.80 | 24.952 | 104.80* |
| Microcrystalline cellulose 102 | 44.048 | 185.00 | 54.048 | 227.00 |
| Pregelatinized starch STARCH 1500 ^{®} | 25.000 | 105.00 | 15.000 | 63.00 |
| Sodium carboxymethyl starch VIVASTAR P | 5.000 | 21.00 | 5.000 | 21.00 |
| Sodium stearyl fumarate | 0.500 | 2.10 | 0.500 | 2.10 |

| Added externally | | | | |
|---|---|---|---|---|
| Sodium stearyl fumarate | 0.500 | 2.10 | 0.500 | 2.10 |
| Total | 100.000 | 420.00 | 100.000 | 420.00 |

| Capsule shell | | | | |
|---|---|---|---|---|
| Gelatin hollow capsule, 0# | 1 capsule | 96.00 | 1 capsule | 96.00 |

**Table 14. Effect of diluent ratio on dissolution results (dissolution method 2)**

| **Experiment No.** | **Dissolution item** | **Dissolution rate (%)** | | | | | |
|---|---|---|---|---|---|---|---|
| | | **5 min** | **15 min** | **30 min** | **45 min** | **60 min** | **75 min** |
| F11 | median | 31 | 81 | 87 | 89 | 90 | 95 |
| | RSD% | 42.2 | 3.8 | 2.6 | 2.1 | 2.1 | 0.8 |
| F12 | median | 37 | 78 | 85 | 88 | 89 | 94 |
| | RSD% | 19.9 | 1.5 | 1.1 | 1.3 | 1.1 | 0.8 |

From the dissolution results, it can be seen that there is no significant difference in the dissolution results of F11 and F12, indicating that the ratio of pregelatinized starch has no significant effect on the dissolution rate. Therefore, it was finally determined to use 25 wt% of pregelatinized starch STARCH 1500 ^{®} to conduct subsequent process development.

### Example 3: Effect of disintegrant and solubilizer on dissolution performance

Based on the experimental results of Example 2, the dissolution of the capsules is used as an indicator to investigate the type of disintegrant. The specific composition of experiments F7 and F10 is shown in Table 15, where F10 changes the type of disintegrant, and the other conditions are the same as in Example 2. The dissolution results are shown in Table 16 and Fig. 3, where dissolution method 2 is used for evaluation.

**Table 15. Specific compositions of F7 and F10**

| **Experiment No.** | **F7** | | **F10** | |
|---|---|---|---|---|
| **Disintegrant type** | **Sodium carboxymethyl starch SD711** | | **Sodium carboxymethyl starch VIVASTAR P** | |
| **Ingredient** | **Percentage %** | **Unit dosage mg** | **Percentage %** | **Unit dosage mg** |
| Added internally | | | | |
| TJ0113 | 24.952 | 104.80* | 24.952 | 104.80* |
| Microcrystalline cellulose 102 | 56.048 | 235.40 | 56.048 | 235.40 |
| Pregelatinized starch STARCH 1500 ^{®} | 15.000 | 63.00 | 15.000 | 63.00 |
| Croscarmellose Sodium SD711 | 3.000 | 12.60 | N/A | N/A |
| Sodium carboxymethyl starch VIVASTAR P | N/A | N/A | 3.000 | 12.60 |
| Sodium stearyl fumarate | 0.500 | 2.10 | 0.500 | 2.10 |

| Added externally | | | | |
|---|---|---|---|---|
| Sodium stearyl fumarate | 0.500 | 2.10 | 0.500 | 2.10 |
| Total | 100.000 | 420.00 | 100.000 | 420.00 |

| Capsule shell | | | | |
|---|---|---|---|---|
| Gelatin hollow capsule, 0# | 1 capsule | 96.00 | 1 capsule | 96.00 |

**Table 16. Effects of disintegrant types on dissolution results (dissolution method 2)**

| **Experiment No.** | **Dissolution item** | **Dissolution rate (%)** | | | | | |
|---|---|---|---|---|---|---|---|
| | | **5 min** | **15 min** | **30 min** | **45 min** | **60 min** | **75 min** |
| F7 | median | 39 | 65 | 81 | 86 | 88 | 95 |
| | RSD% | 10.5 | 5.7 | 2.8 | 2.3 | 2.1 | 0.9 |
| F10 | median | 51 | 78 | 84 | 87 | 88 | 96 |
| | RSD% | 6.2 | 1.5 | 0.6 | 0.9 | 0.8 | 0.1 |

From the above results, it can be seen that the dissolution of F10 in the experiment is slightly higher than that of F7 and has a smaller RSD % before 30 minutes, so the disintegrant type is selected as carboxymethyl starch sodium VIVASTARP.

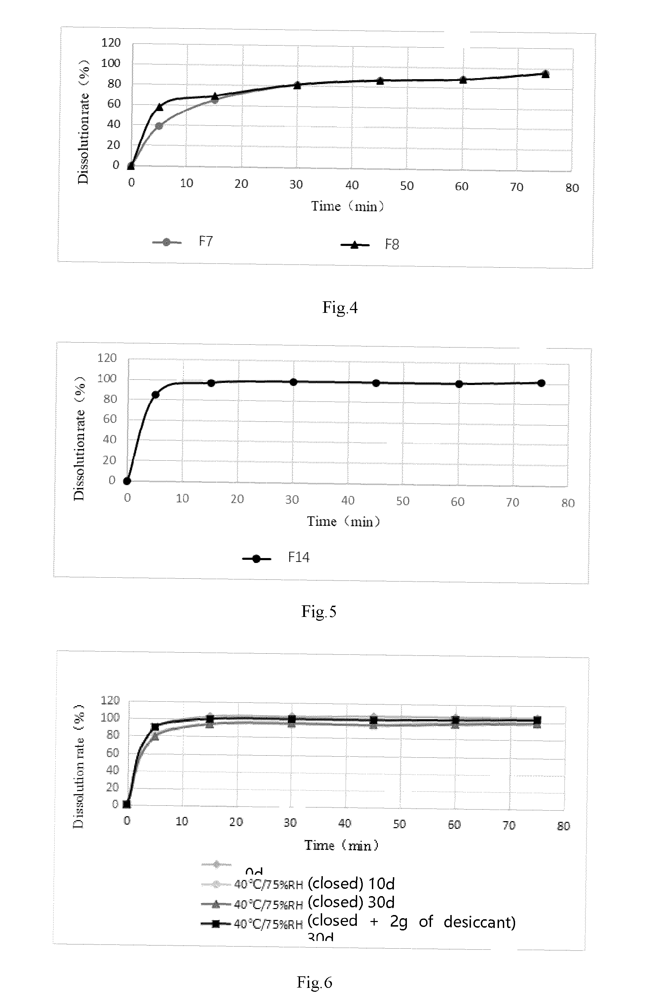

On this basis, the effect of the addition of solubilizer on the dissolution of product is further investigated. The composition of F7 and F8 is shown in Table 17, where a solubilizer is added to F8, and the other conditions are the same as in Example 2. The dissolution results are shown in Table 18 and Fig. 4, where dissolution method 2 is used for evaluation.

**Table 17. Specific composition of F7 and F8**

| **Experiment No.** | **F7** | | **F8** | |
|---|---|---|---|---|
| **Solubilizer** | **NA** | | **Sodium dodecyl sulfate Kolliphor SLS Fine** | |
| **Ingredient** | **Percentage%** | **Unit dosage mg** | **Percentage %** | **Unit dosage mg** |
| Added internally | | | | |
| TJ0113 | 24.952 | 104.80* | 24.952 | 104.80* |
| Microcrystalline cellulose 102 | 56.048 | 235.40 | 53.048 | 222.80 |
| Pregelatinized starch STARCH 1500 ^{®} | 15.000 | 63.00 | 15.000 | 63.00 |
| Sodium dodecyl sulfate Kolliphor SLS Fine | N/A | N/A | 3.000 | 12.60 |
| Croscarmellose Sodium SD711 | 3.000 | 12.60 | 3.000 | 12.60 |
| Sodium stearyl fumarate | 0.500 | 2.10 | 0.500 | 2.10 |

| Added externally | | | | |
|---|---|---|---|---|
| Sodium stearyl fumarate | 0.500 | 2.10 | 0.500 | 2.10 |
| Total | 100.000 | 420.00 | 100.000 | 420.00 |

| Capsule shell | | | | |
|---|---|---|---|---|
| Gelatin hollow capsule, 0# | 1 capsule | 96.00 | 1 capsule | 96.00 |

**Table 18. Effect of solubilizer on dissolution results (dissolution method 2)**

| **Experiment No.** | **Dissolution item** | **Dissolution rate (%)** | | | | | |
|---|---|---|---|---|---|---|---|
| | | **5 min** | **15 min** | **30 min** | **45 min** | **60 min** | **75 min** |
| F7 | median | 39 | 65 | 81 | 86 | 88 | 95 |
| | RSD% | 10.5 | 5.7 | 2.8 | 2.3 | 2.1 | 0.9 |
| F8 | median | 58 | 69 | 81 | 86 | 88 | 95 |
| | RSD% | 12.4 | 9.8 | 0.9 | 1.6 | 2.4 | 1.1 |

From the above results, it can be seen that there is no significant difference in the dissolution results of experiments F7 and F8, indicating that the addition of the solubilizer Sodium dodecyl sulfate Kolliphor SLS Fine has no significant effect on the dissolution rate. Therefore, it is finally determined that Sodium dodecyl sulfate Kolliphor SLS Fine is not used as a solubilizer.

The 20 mg capsule has an equal proportional prescription with 100 mg capsule, and the process is basically the same. The composition and dissolution results of the 20 mg capsule (evaluated using dissolution method 3) are shown in Table 19, Table 20 and Fig. 5.

**Table 19. Specific composition of the 20mg capsule**

| **Experiment No.** | **F14** | |
|---|---|---|
| **Specification** | **20 mg** | |
| **Ingredient** | **Percentage%** | **Unit dosage mg** |
| Added internally | | |
| TJ0113 | 24.952 | 20.96* |
| Microcrystalline cellulose 102 | 44.048 | 37.00 |
| Pregelatinized starch STARCH 1500 ^{®} | 25.000 | 21.00 |
| Sodium carboxymethyl starch VIVASTAR P | 5.000 | 4.20 |
| Sodium stearyl fumarate | 0.500 | 0.42 |

| Added externally | | |
|---|---|---|
| Sodium stearyl fumarate | 0.500 | 0.42 |
| Total | 100.00 | 84.00 |

| Capsule shell | | |
|---|---|---|
| Gelatin hollow capsule, 4# opaque rich yellow (cap color number: 41.813, body color number: 41.813) -CN, US | 1 capsule | 38.00 |
| Remarks: * the specification is based on free acid radical, the molecular weight of free acid radical is 483.33, TJ0113 is sodium salt, its molecular weight is 506.32, and the conversion coefficient is 1.048. | | |

**Table 20. Dissolution result of the 20mg capsule (dissolution method 3)**

| **Experiment No.** | **Dissolution item** | **Dissolution rate (%)** | | | | | |
|---|---|---|---|---|---|---|---|
| | | **5 min** | **15 min** | **30 min** | **45 min** | **60 min** | **75 min** |
| F14 | median | 85 | 97 | 99 | 99 | 99 | 101 |
| | RSD% | 2.3 | 1.4 | 1.2 | 0.6 | 1.3 | 1.6 |

From the above data, it can be seen that the 20mg capsule can be dissolved quickly and completely, which meets expectations, indicating that its specific composition design is reasonable.

### Example 4: Overall stability test of preparation

The process flow of the preparation in this Example is:
API and microcrystalline cellulose 102 pass through a 50-mesh sieve together; the sieved API and microcrystalline cellulose 102 then pass through a 50-mesh sieve twice together with pregelatinized starch STARCH 1500 ^{®}; after sieving, API, microcrystalline cellulose 102, pregelatinized starch STARCH 1500 ^{®} are premixed with sodium carboxymethyl starch VIVASTAR P; sodium stearyl fumarate added internally are added into the premixed materials for main mixing; after main mixing, dry granulation is carried out; after dry granulation, sodium stearyl fumarate added externally are added into the materials for total mixing; finally the total mixed granules are filled into capsules.

The specific process flow and parameters are as follows:

### 1. Weighing

Weighing TJ0113, microcrystalline cellulose 102, and pregelatinized starch STARCH 1500 ^{®}, Sodium carboxymethyl starch VIVASTARP and sodium stearic fumarate according to the prescription amount.

### 2. Sieving

Firstly, passing the microcrystalline cellulose 102 and the raw material through a 50-mesh sieve once together to obtain sieved mixture 1; then passing the sieved mixture 1 and pregelatinized starch STARCH 1500 ^{®} through a 50-mesh sieve twice together; passing sodium carboxymethyl starch VIVASTAR P through a 50-mesh sieve, and passing sodium stearic fumarate added internally and externally through a 30-mesh sieve, respectively.

### 3. Premixing

Placing the raw and auxiliary materials except sodium stearic fumarate into a hopper of mixer to mix for 20 minutes, and the mixing speed is 20rpm.

### 4. Main mixing

After premixing, adding sodium stearic fumarate (added internally) into the hopper, and starting the main mixing for 5 minutes, the mixing speed is 20 rpm.

### 5. Dry granulation and sizing

Placing the main mixed powder in a dry granulator for granulating, the granulation parameters are: feeding speed: 28rpm, roller pressure: 30kg/cm², roller speed: 8rpm. Crushing the strip obtained by dry granulation, the secondary sizing screen is a circular screen with 0.8mm pore, and the sizing speed is 150rpm.

### 6. Total mixing

Mixing the granulated dry granules and sieved sodium stearic fumarate (added externally) in the hopper of mixer for 5 minutes, and the mixing speed is 20rpm.

### 7. Capsule filling

Placing the total mixed granules in a capsule filler for capsule filling, filling into the 4# gelatin hollow capsule shell for 20 mg specification, and filling into the 0# gelatin hollow capsule shell for 100 mg specification.

### Preliminary stability investigation in the laboratory

In order to investigate the rationality and stability of the preparation prototype prescription, a preliminary stability investigation was conducted on laboratory samples.

The sample batches used for stability investigation are F14 and F13, the specific composition and sample information are shown in Tables 21 and 22.

**Table 21. Specific composition of F14 and F13**

| **Experiment No.** | | **F14** | **F13** |
|---|---|---|---|
| **Specification** | | **20 mg** | **100 mg** |
| **Batch Quantity** | | **4,000 capsules** | **1,800 capsules** |
| **Ingredient** | **Percentage%** | **mg/capsule** | **mg/capsule** |
| Added internally | | | |
| TJ0113 | 24.952 | 20.96^{*} | 104.80^{*} |
| Microcrystalline cellulose 102 | 44.048 | 37.00 | 185.00 |
| Pregelatinized starch STARCH 1500 ^{®} | 25.000 | 21.00 | 105.00 |
| Sodium carboxymethyl starch VIVASTAR P | 5.000 | 4.20 | 21.00 |
| Sodium stearyl fumarate | 0.500 | 0.42 | 2.10 |

| Added externally | | | |
|---|---|---|---|
| Sodium stearyl fumarate | 0.500 | 0.42 | 2.10 |
| Total | 100.000 | 84.00 | 420.00 |

| Capsule shell | | | |
|---|---|---|---|
| Gelatin hollow capsule, 4# | 1 capsule | 38.00 | N/A |
| Gelatin hollow capsule, 0# | 1 capsule | N/A | 96.00 |
| Remarks: * the specification is based on free acid radical, the molecular weight of free acid radical is 483.33, TJ0113 is sodium salt, its molecular weight is 506.32, and the conversion coefficient is 1.048. | | | |

**Table 22. Sample information for stability test**

| Sample name | **TJ0113 capsule, 20 mg** | **TJ0113 capsule, 100 mg** |
|---|---|---|
| Batch No. | F14 | F13 |
| Production Date | 2022.10.20 | 2022.10.20 |
| Specification | 36 capsules/bottle | 36 capsules/bottle |
| Sample package | Oral solid medicinal high density polyethylene bottle 45mL | Oral solid medicinal high density polyethylene bottle 75mL |
| | Oral solid medicinal polypropylene children's safety combination bottle cap CRC33-4 | Oral solid medicinal polypropylene children's safety combination bottle cap CRC33-4 |
| | Medicinal solid polyethylene bottled silica gel desiccant 1g/bag-English printing | Medicinal solid polyethylene bottled silica gel desiccant 1g/bag-English printing |

The placement conditions of the samples F13 and F14 of the two specifications are the same, see Table 23 for the specific placement conditions.

**Table 23. Sample placement conditions for stability test**

| **Placement conditions** | **10d** | **30d** | **Backup** |
|---|---|---|---|
| 40°C/75%RH (opened) | 1 bottle | 1 bottle | N/A |
| 40°C/75%RH (closed) | 1 bottle | 1 bottle | N/A |
| 40°C/75%RH (closed+1g of desiccant) | 1 bottle | 1 bottle | N/A |
| 40°C/75%RH (closed+2g of desiccant) | N/A | N/A | 1 bottle |
| 30°C/65%RH (closed+1g of desiccant) | 1 bottle | 1 bottle | N/A |
| 25°C/60%RH (closed+1g of desiccant) | 1bottle | 1 bottle | N/A |
| 2-8°C (closed+1g of desiccant) | 1 bottle | 1 bottle | N/A |

The dissolution results of stability investigation are shown in Table 24 and Figs. 6 and 7, and the relevant substance results of stability investigation are shown in Table 25.

**Table 24. Dissolution results of stability of 20mg and 100mg capsules (evaluated using dissolution method 3)**

| **Experiment No.** | **Placement conditions and time** | | **Item** | **Dissolution results (%) (n=3)** | | | | | |
|---|---|---|---|---|---|---|---|---|---|
| | | | | **5 min** | **15 min** | **30 min** | **45 min** | **60 min** | **75 min** |
| F14 | 0d | | median | 90 | 103 | 104 | 105 | 105 | 105 |
| | | | RSD% | 3.9 | 2.8 | 3.3 | 2.7 | 3.1 | 3.1 |
| | 40°C/75%RH (closed) | 10d | median | 81 | 95 | 96 | 96 | 98 | 98 |
| | | | RSD% | 4.2 | 3.8 | 3.7 | 4.9 | 4.3 | 3.2 |
| | | 30d | median | 79 | 94 | 96 | 95 | 97 | 99 |
| | | | RSD% | 9.6 | 3.4 | 3.0 | 2.5 | 2.8 | 3.6 |
| | 40°C/75%RH (closed + 2g of desiccant) | 30d | median | 90 | 100 | 101 | 101 | 102 | 103 |
| | | | RSD% | 7.8 | 4.2 | 4.5 | 4.5 | 4.6 | 3.1 |
| F13 | 0d | | median | 63 | 93 | 94 | 95 | 95 | 97 |
| | | | RSD% | 16.9 | 3.2 | 4.4 | 3.2 | 2.7 | 3.7 |
| | 40°C/75%RH (closed) | 10d | median | 65 | 98 | 99 | 100 | 99 | 101 |
| | | | RSD% | 26.2 | 2.1 | 2.2 | 2.1 | 1.9 | 1.8 |
| | | 30d | median | 64 | 94 | 97 | 98 | 97 | 100 |
| | | | RSD% | 5.5 | 4.1 | 2.7 | 2.3 | 2.6 | 1.0 |
| | 40°C/75%RH (closed + 2g of desiccant) | 30d | median | 70 | 97 | 98 | 99 | 99 | 101 |
| | | | RSD% | 2.2 | 2.5 | 2.1 | 2.0 | 1.8 | 0.8 |

Results analysis: From the above data, compared with the 0d dissolution data, there is no significant change in the dissolution of 20 mg and 100 mg capsules after 30 days of placement under the conditions of 40°C/75% RH (closed) and 40°C/75% RH (closed + 2g of desiccant).

**Table 25. Relevant substance results of stability of 20mg and 100mg capsules (%)**

| **Specific ation** | **Placement conditions** | **Days** | **RRT0.47 /0.46** | **RRT0.87** | **RRT1.16** | **RRT1.21 /1.20** | **RRT1.30 /1.29** | **RRT1.33 /1.34** | **RRT1.37 /1.38** | **RRT1.42 /1.41** | **RRT1.82 /1.73** | **RRT1.83 /1.74** | **Total** |
|---|---|---|---|---|---|---|---|---|---|---|---|---|---|
| 20 mg | - | 0d | <0.05 | <0.05 | 0.85 | 0.08 | 0.11 | 0.09 | <0.05 | <0.05 | <0.05 | 0.05 | 1.18 |
| | 40°C/75%RH (closed) | 10d | <0.05 | <0.05 | 0.86 | <0.05 | 0.09 | <0.05 | 0.05 | <0.05 | <0.05 | <0.05 | 1.00 |
| | | 30d | 0.06 | 0.05 | 0.90 | 0.06 | 0.12 | 0.07 | 0.10 | <0.05 | 0.10 | 0.05 | 1.51 |
| | 40°C/75%RH (closed + 1g of desiccant) | 10d | <0.05 | <0.05 | 0.85 | <0.05 | 0.09 | 0.05 | <0.05 | <0.05 | ND | <0.05 | 0.99 |
| | | 30d | <0.05 | <0.05 | 0.84 | 0.06 | 0.10 | 0.08 | 0.09 | <0.05 | <0.05 | 0.05 | 1.22 |
| | 40°C/75%RH (closed + 2g of desiccant) | 30d | <0.05 | <0.05 | 0.84 | 0.06 | 0.09 | 0.08 | 0.08 | <0.05 | <0.05 | 0.05 | 1.20 |
| | 25°C/60%RH (closed + 1g of desiccant) | 10d | <0.05 | <0.05 | 0.85 | 0.05 | 0.07 | <0.05 | <0.05 | <0.05 | ND | <0.05 | 0.97 |
| | | 30d | <0.05 | <0.05 | 0.84 | 0.07 | 0.07 | 0.09 | 0.07 | <0.05 | <0.05 | <0.05 | 1.14 |
| | 2-8°C (closed + 1g of desiccant) | 30d | <0.05 | <0.05 | 0.83 | 0.08 | 0.05 | 0.09 | 0.08 | <0.05 | <0.05 | <0.05 | 1.13 |
| 100 mg | - | 0d | <0.05 | <0.05 | 0.85 | 0.10 | 0.09 | 0.09 | <0.05 | <0.05 | <0.05 | 0.05 | 1.18 |
| | 40°C/75%RH (closed) | 10d | <0.05 | <0.05 | 0.85 | <0.05 | 0.09 | <0.05 | 0.05 | <0.05 | <0.05 | <0.05 | 0.99 |
| | | 30d | 0.05 | <0.05 | 0.86 | 0.06 | 0.10 | 0.08 | 0.11 | <0.05 | 0.06 | 0.05 | 1.37 |
| | 40°C/75%RH (closed + 1g of desiccant) | 10d | <0.05 | <0.05 | 0.84 | <0.05 | 0.11 | 0.05 | 0.05 | <0.05 | <0.05 | <0.05 | 1.05 |
| | | 30d | 0.05 | <0.05 | 0.85 | 0.06 | 0.10 | 0.08 | 0.09 | <0.05 | <0.05 | 0.05 | 1.28 |
| | 40°C/75%RH (closed + 2g of desiccant) | 30d | <0.05 | <0.05 | 0.84 | 0.06 | 0.10 | 0.08 | 0.09 | <0.05 | <0.05 | 0.05 | 1.22 |
| | 25°C/60%RH (closed + 1g of desiccant) | 10d | <0.05 | <0.05 | 0.83 | 0.05 | 0.07 | 0.05 | <0.05 | <0.05 | ND | <0.05 | 1.00 |
| | | 30d | <0.05 | <0.05 | 0.84 | 0.07 | 0.06 | 0.09 | 0.07 | <0.05 | <0.05 | <0.05 | 1.13 |
| | 2-8°C (closed + 1g of desiccant) | 30d | <0.05 | <0.05 | 0.83 | 0.08 | 0.05 | 0.09 | 0.06 | <0.05 | <0.05 | <0.05 | 1.11 |

Results Analysis: After 20mg capsules were placed under the condition of 40°C/75% RH (closed) for 30 days, the relevant substances increased at RRT = 1.16, RRT = 1.37/1.38 and RRT = 1.82/1.73. After 100mg capsules were placed under the condition of 40°C/75% RH (closed) for 30 days, the relevant substances increased at RRT = 1.37/1.38. After 20mg and 100mg capsules were placed under other conditions for 30 days, there was no significant change in the relevant substances.

Based on the dissolution and relevant substance results, it is finally determined that the product package is 45mL and 75mL oral solid medicinal high-density polyethylene bottles and oral solid medicinal polypropylene children's safety combination bottle cap with aluminum foil seal plus two 1g of silicone desiccants filled in medicinal solid paper bag with English printing, the storage conditions are "protect from light, store in a tightly closed container at 2-8°C".

### Example 5: Effect of dry granulation on the crystal form of raw material

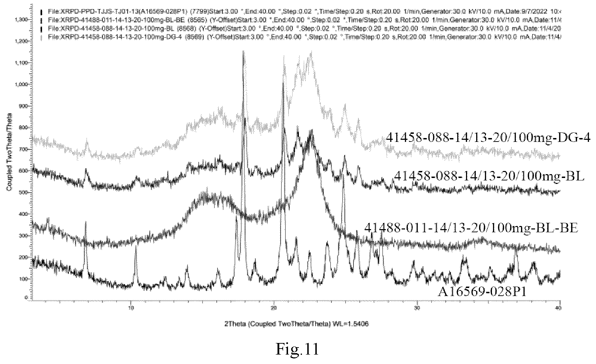

In this test example, the effect of dry granulation on the crystal form of the raw material is investigated, the prescription composition is shown in F13 in Table 21, and the XRPD results are shown in Fig. 9. The operation of XRPD is the same as that of the preparation example. The obtained XRPD pattern is shown in Fig. 11, wherein curve A16569-028P1 represents the raw material; curve 41458-088-14/13-20/100mg-BL represents the mixing powder before dry granulation; curve 41488-011-14/13-20/100mg-BL-BE represents the blank auxiliary material before dry granulation; curve 41458-088-14/13-20/100mg-DG-4 represents the dry granules after dry granulation. From the above curves, it can be seen that the main characteristic peaks of the XRPD pattern of the intermediate active samples before and after dry granulation did not change significantly, thus it is preliminarily judged that the dry granulation process has no effect on the crystal form of the raw material.

## Claims

1. A pharmaceutical composition, **characterized in that** the pharmaceutical composition contains a compound of Formula I or a pharmaceutically acceptable salt thereof, a diluent, a disintegrant, a lubricant, and does not contain any of the following ingredients: hydroxypropyl cellulose, crospovidone, colloidal silica, magnesium stearate, talc, mannitol, croscarmellose sodium, 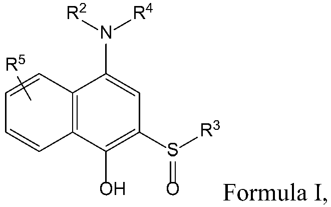
wherein, R² is hydrogen, C_{1~4} alkyl, C_{3~6} cycloalkyl, four to six-membered epoxy alkyl;
R³ is 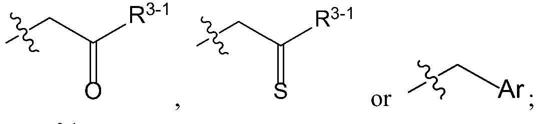
wherein, R³⁻¹ is hydrogen, hydroxyl, C_{1~4} alkyl, C_{1~4} alkoxy, C_{3~6} cycloalkyl, three to six-membered epoxy alkyl, -N(R³⁻²R^{3-2a}), -CH₂C(O)R³⁻², -CH₂C(O)OR³⁻², -CH₂C(O)NR³⁻² R^{3-2a}
R³⁻² and R^{3-2a} are independently hydrogen, C_{1~4} alkyl or three to six-membered cycloalkyl respectively,
Ar is phenyl, 5 or 6-membered monocyclic heteroaryl, 5 or 6-membered monocyclic heteroaryl substituted by at least one R³⁻³, said R³⁻³ is hydrogen, halogen, C_{1~4} alkyl, three to six-membered cycloalkyl, hydroxyl, C_{1~4} Alkoxy, three to six-membered epoxy alkyl, C_{1~4} halogenated alkyl, C_{2~4} alkenyl, C_{2~4} alkynyl, -N(R^{3-3a}R^{3-3b}) or phenyl,
R^{3-3a} and R^{3-3b} are independently hydrogen, C_{1~4} alkyl or three to six-membered cycloalkyl respectively;
R⁴ is 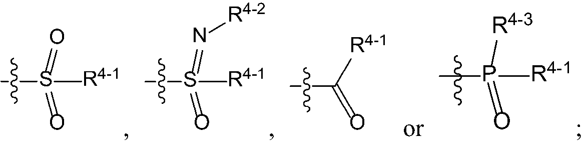
wherein, R⁴⁻¹ is phenyl, phenyl substituted by at least one R⁴⁻¹¹, 5 or 6-membered monocyclic heteroaryl, 5 or 6-membered monocyclic heteroaryl substituted by at least one R⁴⁻¹¹, 8 to 10-membered fused bicyclic heteroaryl, 8 to 10-membered fused bicyclic heteroaryl substituted by at least one R⁴⁻¹¹, said R⁴⁻¹¹ is halogen, nitro, C_{1~4} alkyl, C_{3~b} cycloalkyl, C_{1~4} alkoxy, -N(R^{4-1a}R^{4-1b}), phenyl, C_{1~4} halogenated alkyl, C_{1~4} halogenated alkoxy or 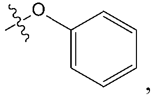
R^{4-1a} and R^{4-1b} are independently hydrogen , C_{1~4} alkyl or C_{3~6} cycloalkyl respectively, and R^{4-1a} and R^{4-1b} may be bonded to each other to form a ring,
R⁴⁻² is C_{1~4} alkyl, C_{3~6} cycloalkyl, or when R² is C_{1~4} alkyl, R⁴⁻² is bonded to R² to form a 4~8-membered ring,
R⁴⁻³ is C_{1~4} alkyl or C_{1~4} alkoxy;
the number of R⁵ is 0~5, when the number of R⁵ is not 0, each R⁵ is independently selected from halogen, nitro, nitrile, -N⁺(R⁵⁻¹)₃, C_{1~4} halogenated alkyl, -C(O)OR⁵⁻¹, -C(O)R⁵⁻¹, -C(O)N(R⁵⁻¹R^{5-1a}), -C(O)N(R⁵⁻¹R^{5-1a}), -S(O)₂R⁵⁻¹, -S(O)R⁵⁻¹, -N=C(R⁵⁻¹R^{5-1a}), hydroxyl, C_{1~4} alkyl, phenyl, phenyl with at least one hydrogen substituted by R⁵⁻¹, C_{1~4} alkoxy, -N(R⁵⁻¹R^{5-1a}), -N(R⁵⁻¹)C(O)R^{5-1a}, -OC(O)R⁵⁻¹, -OC(O) N(R⁵⁻¹R^{5-1a}) or -SR⁵⁻¹, wherein R⁵⁻¹, R^{5-1a} and R^{5-1b} are independently hydrogen, C_{1~4} alkyl, C_{2~4} alkenyl, C_{2~4} alkynyl, C_{1~4} alkyl with at least one hydrogen substituted by halogen, C_{2~4} alkenyl with at least one hydrogen substituted by halogen or C_{2~4} alkynyl with at least one hydrogen substituted by halogen respectively.

2. The pharmaceutical composition of claim 1, wherein the compound of Formula I is TJ0113:

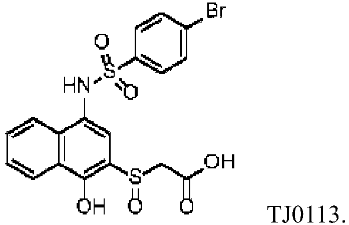

3. The pharmaceutical composition of claim 2, wherein the TJ0113 is in a solid form of a sodium salt represented by the following formula: 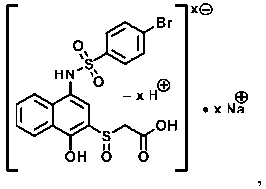 wherein, X is 0.1-2, for example 1.

4. The pharmaceutical composition of claim 3, wherein the X-ray powder diffraction pattern of the solid form exhibits characteristic peaks (Cu Kα rays) at the following 2θ angles: 7.06° (±0.2°) and 20.87° (±0.2°).

5. The pharmaceutical composition according to any one of claims 1-4, wherein the pharmaceutical composition contains a compound TJ0113 or a pharmaceutically acceptable salt thereof, microcrystalline cellulose, pregelatinized starch, sodium carboxymethyl starch, sodium stearyl fumarate.

6. The pharmaceutical composition of claim 5, wherein the pharmaceutical composition consists of a compound TJ0113 or a pharmaceutically acceptable salt thereof, microcrystalline cellulose, pregelatinized starch, sodium carboxymethyl starch, sodium stearyl fumarate.

7. The pharmaceutical composition according to any one of claims 1-4, wherein a content percentage of the compound of Formula I or the pharmaceutically acceptable salt thereof in the pharmaceutical composition is 1.0% to 50% by weight, a content percentage of diluent in the pharmaceutical composition is 30% to 90% by weight, a content percentage of disintegrant in the pharmaceutical composition is 0.1% to 25% by weight, and a content percentage of lubricant in the pharmaceutical composition is 0.01% to 5.0% by weight.
